Europäisches Patentamt

European Patent Office

Office européen des brevets

(11) Veröffentlichungsnummer: **0 264 795**
A2

(12) # EUROPÄISCHE PATENTANMELDUNG

(21) Anmeldenummer: **87114975.3**

(22) Anmeldetag: **13.10.87**

(51) Int. Cl.⁴: **C07K 5/00** , A61K 37/02 , C07C 127/17 , C07D 233/64 , A61K 31/17

(30) Priorität: **22.10.86 DE 3635907**

(43) Veröffentlichungstag der Anmeldung: **27.04.88 Patentblatt  88/17**

(84) Benannte Vertragsstaaten: **AT BE CH DE ES FR GB IT LI NL SE**

(71) Anmelder: **Merck Patent Gesellschaft mit beschränkter Haftung Frankfurter Strasse 250 D-6100 Darmstadt(DE)**

(72) Erfinder: **Raddatz, Peter, Dr. Grafenstrasse 37 D-6100 Darmstadt(DE)** Erfinder: **Schmitges, Claus J., Dr. Karolinger Strasse 5 D-6114 Gross-Umstadt(DE)** Erfinder: **Minck, Klaus Otto, Dr. Büchestrasse 8 D-6105 Ober-Ramstadt(DE)**

(54) **Aminosäurederivate.**

(57)  Neue Aminosäurederivate der Formel I
X-Z-NR²-CHR³-CHOH-(CH₂)ₙ-NR⁴-E-Y     I
worin
X, Z, R², R³, R⁴, E, Y und n die in Patentanspruch 1 angegebene Bedeutung haben,
sowie ihre Salze hemmen die Aktivität des menschlichen Plasmarenins.

EP 0 264 795 A2

# Aminosäurederivate

Die Erfindung betrifft neue Aminosäurederivate der Formel I

$$X\text{-}Z\text{-}NR^2\text{-}CHR^3\text{-}CHOH\text{-}(CH_2)_n\text{-}NR^4\text{-}E\text{-}Y \qquad I$$

worin

X  H, $R^1\text{-}O\text{-}C_mH_{2m}\text{-}CO\text{-}$, $R^1\text{-}C_mH_{2m}\text{-}O\text{-}CO\text{-}$, $R^1\text{-}C_mH_{2m}\text{-}CO\text{-}$, $R^1\text{-}SO_2\text{-}$ oder $(R^1\text{-}C_mH_{2m})\text{-}L(R^1\text{-}C_pH_{2p})\text{-}C_rH_{2r}\text{-}CO\text{-}$,

Z 1 bis 4 peptidartig miteinander verbundene Aminosäurereste ausgewählt aus der Gruppe bestehend aus Abu, Ada, Ala, Arg, Asn, Asp, Bia, Cal, Dab, Gln, Glu, Gly, His, Hph, N(im)-Alkyl-His, Ile, Leu, tert.-Leu, Lys, Met, αNal, βNal, Nbg, Nle, Orn, Phe, Pro, Pyr, Ser, Thr, Tic, Trp, Tyr und Val,

E  -CONH-, -CSNH-, -COO-, -SO₂-, -SO₂NH- oder -PO(OA)-O-,

Y  $R^5$, $\text{-}(CHR^5)_s\text{-}COOR^6$ oder $\text{-}(CHR^5)_s\text{-}CONR^7R^8$,

$R^1$, $R^3$, $R^6$, $R^7$ und $R^8$ jeweils H, A, Ar, Ar-alkyl, Het, Het-alkyl, unsubstituiertes oder ein-oder mehrfach durch A, AO und/oder Hal substituiertes Cycloalkyl mit 3-7 C-Atomen, Cycloalkylalkyl mit 4-11 C-Atomen, Bicycloalkyl der Tricycloalkyl mit jeweils 7-14 C-Atomen, Bicycloalkylalkyl oder Tricycloalkylalkyl mit jeweils 8-18 C-Atomen,

$R^2$ und $R^4$ jeweils H oder A,

$R^5$ H, A, Ar, Ar-alkyl, Cycloalkyl mit 3-7 C-Atomen oder Cycloalkylalkyl mit 4-11 C-Atomen,

L CH oder N,

m, p und r jeweils 0, 1, 2, 3, 4 oder 5,

n 1 oder 2,

s 0 oder 1

Ar unsubstituiertes oder ein-oder mehrfach durch A, AO, Hal, CF₃, OH und/oder NH₂ substituiertes Phenyl oder unsubstituiertes Naphthyl,

Het einen gesättigten oder ungesättigten 5-oder 6-gliedrigen heterocyclischen Rest mit 1-4 N-, O-und/oder S-Atomen, der mit einem Benzolring kondensiert und/oder ein-oder mehrfach durch A, AO, Hal, CF₃, HO, O₂N, Carbonylsauerstoff, H₂N, HAN, A₂N, AcNH, AS, ASO, ASO₂, AOOC, CN, H₂NCO, H₂NSO₂, ASO₂NH, Ar, Ar-alkenyl, Hydroxyalkyl und/oder Aminoalkyl mit jeweils 1-8 C-atomen substituiert sein kann und/oder dessen N-und/oder S-Heteroatome auch oxydiert sein können,

Hal F, Cl, Br oder J,

Ac A-CO-, Ar-CO-oder A-NH-CO-,

-alkyl-eine Alkylengruppe mit 1-8 C-Atomen und

A Alkyl mit 1-8 C-Atomen bedueten,

E-Y auch Pyrrolidinocarbonyl, Piperidinocarbonyl, Morpholinocarbonyl, Pyrrolidinosulfonyl, Piperidinosulfonyl oder Morpholinosulfonyl bedeuten kann,

worin ferner an S elle einer oder mehrerer -NH-CO-Gruppen auch eine oder mehrere -NA-CO-Gruppen stehen können,

sowie deren Salze.

Ähnliche Verbindungen sind aus EP-A-77028, EP-A-155809, EP-A-156321, EP-A-156322 und EP-A-163237 bekannt, insbesondere jedoch aus US-A-4599198.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem hemmen sie die Aktivität des menschlichen Plasmarenins. Diese Wirkung kann z. B. nach der Methode von F. Fyhrquist et. al., Clin.Chem. 22, 250-256 (1976), nachgewiesen werden. Bemerkenswert ist, daß diese Verbindungen sehr spezifische Hemmer des Renins sind; für die Hemmung anderer Aspartylproteinasen (z. B. Pepsin und Kathepsin D) sind in der Regel wesentlich höhere Konzentrationen dieser Verbindungen notwendig.

Die Verbindungen können als Arzneimittelwirkstoffe in der Human-und Veterinärmedizin eingesetzt werden, insbesondere zur Prophylaxe und zur Behandlung von Herz-, Kreislauf-und Gefäßkrankheiten, vor allem von Hypertonie, Herzinsuffizienz und Hyperaldosteronismus. Außerdem können die Verbindungen zu diagnostischen Zwecken verwendet werden, um bei Patienten mit Hypertonie oder Hyperaldosteronismus den möglichen Beitrag der Reninaktivität zur Aufrechterhaltung des pathologischen Zustands zu bestimmen.

Die vor-und nachstehend aufgeführten Abkürzungen von Aminosäureresten stehen für die Reste -NH-CHR-CO-(worin R die für jede Aminosäure bekannte spezifische Bedeutung hat) folgender Aminosäuren:

Abu 2-Aminobuttersäure
Ada Adamantylalanin
Ala Alanin
Arg Arginin
Asn Asparagin
Asp Asparaginsäure
Bia Benzimidazolylalinin
Cal Cyclohexylalanin
Dab 2,4-Diaminobuttersäure
Gln Glutamin
Glu Glutaminsäure
Gly Glycin
His Histidin
N(im)-Alkyl-His in 1-Stellung des Imidazolrings durch A substituiertes Histidin
Hph Homo-phenylalanin (2-Amino-4-phenylbuttersäure)
Ile Isoleucin

Leu Leucin
tert.-Leu tert.-Leucin
Lys Lysin
Met Methionin
αNal α-Naphthylalanin
βNal β-Naphthylalanin
Nbg (2-Norbornyl)-glycin
Nle Norleucin
N-Me-His N-Methyl-histidin
N-Me-Phe N-Methyl-phenylalanin
Orn Ornithin
Phe Phenylalanin
Pro Prolin
Pyr Pyridylalanin
Ser Serin
Thr Threonin
Tic Tetrahydroisochinolin-1-carbonsäure
Trp Tryptophan
Tyr Tyrosin
Val Valin.

Ferner bedeutet nachstehend:

BOC tert.-Butoxycarbonyl
imi-BOM Benzyloxymethyl in 1-Stellung des Imidazolrings
CBZ Benzyloxycarbonyl
DNP 2,4-Dinitrophenyl
imi-DNP 2,4-Dinitrophenyl in 1-Stellung des Imidazolrings
ETNC N-Ethylcarbamoyl
ETOC Ethoxycarbonyl
FMOC 9-Fluorenylmethoxycarbonyl
IPNC N-Isopropylcarbamoyl
IPOC Isopropoxycarbonyl
MC Morpholinocarbonyl
OMe Methylester
OEt Ethylester
PBB 4-Phenyl-2-benzylbutyryl
POA Phenoxyacetyl
DCCI Dicyclohexylcarbodiimid
HOBt 1-Hydroxybenzotriazol.

Sofern die vorstehend genannten Aminosäuren in mehreren enantiomeren Formen auftreten können, so sind vor-und nachstehend, z. B. als Bestandteil der Verbindungen der Formel I, alle diese Formen und auch ihre Gemische (z. B. die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich etwas anderes angegeben ist.

Vor-und nachstehend haben die Reste bzw. Parameter X, Z, E, Y, D, $R^1$ bis $R^9$, L, m, n, p, r, Ar, Het, Hal, Ac, -alkyl-, A, $G^1$, $G^2$, $Z^1$ und $Z^2$ die bei den Formeln I, II oder III angegebenen Bedeutungen, falls nicht ausdrücklich etwas anderes angegeben ist. Falls zwei Reste $R^1$ in einer Verbindung der Formel I vorhanden sind, können sie gleich oder voneinander verschieden sein.

In den vorstehenden Formeln hat A 1 - 8, vorzugsweise 1,2,3 oder 4 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2-oder 3-Methylbutyl, 1,1-, 1,2-oder 2,2-Dimethylpropyl, 1-Ethylpropyl, Hexyl, 1-, 2-, 3-oder 4-Methylpentyl, 1,1-, 1,2-, 1,3-, 2,2-, 2,3-oder 3,3-Dimethylbutyl, 1-oder 2-Ethylbutyl, 1-Ethyl-1-methylpropyl, 1-Ethyl-2-methylpropyl, 1,1,2-oder 1,2,2-Trimethylpropyl, Heptyl, Octyl.

Cycloalkyl bedeutet vorzugsweise Cyclopropyl, Cyclobutyl, Cylopentyl, Cyclohexyl oder Cycloheptyl, aber auch z. B. 1-, 2-oder 3-Methylcyclopentyl, 1-, 2-, 3-oder 4-Methylcyclohexyl.

Dementsprechend bedeutet Cycloalkyl-alkyl vorzugsweise Cyclopropylmethyl, 2-Cyclopropylethyl, Cyclobutylmethyl, 2-Cyclobutylethyl, Cyclopentylmethyl, 2-Cyclopentylethyl, Cyclohexylmethyl, 2-Cyclohexylethyl, aber auch z. B. 1-, 2-oder 3-Methylcyclopentylmethyl, 1-, 2-, 3-oder 4-Methylcyclohexylmethyl.

Bicycloalkyl bedeutet vorzugsweise 1-oder 2-Dekalyl, 2-Bicyclo[2,2,1]heptyl oder 6,6-Dimethyl-2-bicyclo[3,1,1]heptyl.

Tricycloalkyl bedeutet vorzugsweise 2-Adamantyl.

Hal bedeutet vorzugsweise F, Cl oder Br, aber auch J.

Ac bedeutet vorzugsweise A-CO-wie Acetyl, Propionyl oder Butyryl, Ar-CO-wie Benzoyl, o-, m-oder p-Methoxybenzoyl oder 3,4-Dimethoxybenzoyl, A-NH-CO-wie N-Methyl-oder N-Ethylcarbamoyl.

Ar bedeutet vorzugsweise Phenyl, ferner bevorzugt o-, m-oder p-Tolyl, o-, m-oder p-Ethylphenyl, o-, m-oder p-Methoxyphenyl, o-, m-oder p-Fluorphenyl, o-, m-oder p-Chlorphenyl, o-, m-oder p-Bromphenyl, o-, m-oder p-Jodphenyl, o-, m-oder p-Trifluormethylphenyl, o-, m-oder p-Hydroxyphenyl, 2,3-, 2,4-, 2,5-, 2,6-, 3,4-oder 3,5-Dimethoxyphenyl, 3,4,5-Trimethoxyphenyl, o-, m-oder p-Aminophenyl, 1-oder 2-Naphthyl.

Dementsprechend bedeutet Ar-alkyl vorzugsweise Benzyl, 1-oder 2-Phenylethyl, o-, m-oder p-Methylbenzyl, 1-oder 2-o-, -m-oder -p-Tolylethyl, o-, m-oder p-Ethylbenzyl, 1-oder 2-o-, -m-oder -p-Ethylphenylethyl, o-, m-oder p-Methoxybenzyl, 1-oder 2-o-, -m-oder -p-Methoxyphenylethyl, -o-, m-oder p-Fluorbenzyl, 1-oder 2-o-, -m-oder -p-Fluorphenylethyl, o-, m-oder p-Chlorbenzyl, 1-oder 2-o-, -m-oder -p-Chlorphenylethyl, o-, m-oder p-Brombenzyl, 1-oder 2-o-, -m-oder -p-Bromphenylethyl, o-, m-oder p-Jodbenzyl, 1-oder 2-o-, -m-oder -p-Jodphenylethyl, o-, m-oder p-Trifluormethylbenzyl, o-, m-oder p-Hydroxybenzyl, 2,3-, 2,4-, 2,5-, 2,6-, 3,4-oder 3,5-Dimethoxybenzyl, 3,4,5-Trimethoxybenzyl, o-, m-oder p-Aminobenzyl, 1-oder 2-Naphthylmethyl.

Het ist vorzugsweise 2-oder 3-Furyl, 2-oder 3-Thienyl, 1-, 2-oder 3-Pyrryl, 1-, 2-, 4-oder 5-Imidazolyl, 1-, 3-, 4-oder 5-Pyrazolyl, 2-, 4-oder 5-Oxazolyl, 3-, 4-oder 5-Isoxazolyl, 2-, 4-oder 5-Thiazolyl, 3-, 4-oder 5-Isothiazolyl, 2-, 3-oder 4-Pyridyl, 2-, 4-, 5-oder 6-Pyrimidyl, weiterhin bevorzugt 1,2,3-Triazol-1-, -4-oder -5-yl, 1,2,4-Triazol-1-, -3-oder -5-yl, 1-oder 5-Tetrazolyl, 1,2,3-Oxadiazol-4-oder -5-yl, 1,2,4-Oxadiazol-3-oder -5-yl, 1,3,4-Thiadiazol-2-oder -5-yl, 1,2,4-Thiadiazol-3-oder -5-yl, 2,1,5-Thiadiazol-3-oder -4-yl, 2-, 3-, 4-, 5-oder 6-2H-Thiopyranyl, 2-, 3-oder 4-4H-Thiopyranyl, 3-oder 4-Pyridazinyl, Pyrazinyl, 2-, 3-, 4-, 5-, 6-oder 7-Benzofuryl, 2-, 3-, 4-, 5-, 6-oder 7-Benzothienyl, 1-, 2-, 3-, 4-, 5-, 6-oder 7-Indolyl, 1-, 2-, 3-, 4-, 5-, 6-oder 7-Isoindolyl, 1-, 2-, 4-oder 5-Benzimidazolyl, 1-, 3-, 4-, 5-, 6-oder 7-Benzopyrazolyl, 2-, 4-, 5-, 6-oder 7-Benzoxazolyl, 3-, 4-, 5-, 6-oder 7-Benzisoxazolyl, 2-, 4-, 5-, 6-oder 7-Benzthiazolyl, 2-, 4-, 5-, 6-oder 7-Benzisothiazolyl, 4-, 5-, 6-oder 7-Benz-2,1,3-oxadiazolyl, 2-, 3-, 4-, 5-, 6-, 7-, oder 8-Chinolyl, 1-, 3-, 4-, 5-, 6-, 7-oder 8-Isochinolyl, 1-, 2-, 3-, 4-oder 9-Carbazolyl, 1-, 2-, 3-, 4-, 5-, 6-, 7-, 8-oder 9-Acridinyl, 3-, 4-, 5-, 6-, 7-oder 8-Cinnolyl, 2-, 4-, 5-, 6-, 7-oder 8-Chinazolyl. Die heterocyclischen Reste konnen auch teilweise oder vollständig hydriert sein. Het kann also z. B. auch bedeuten 2,3-Dihydro-2-, -3-, -4-oder -5-furyl, 2,5-Dihydro-2-, -3-, -4-oder -5-furyl, Tetrahydro-2-oder -3-furyl, Tetrahydro-2-oder -3-thienyl, 2,3-Dihydro-1-, -2-, -3-, -4-oder -5-pyrryl, 2,5-Dihydro-1-, -2-, -3-, -4-oder -5-pyrryl, 1-, 2-oder 3-Pyrrolidinyl, Tetrahydro-1-, -2-oder -4-imidazolyl, 2,3-Dihydro-1-, -2-, -3-, -4-oder -5-pyrazolyl, 2,5-Dihydro-1-, -2-, -3-, -4-oder -5-pyrazolyl, Tetrahydro-1-, -3-, oder -4-pyrazolyl, 1,4-Dihydro-1-, -2-, -3-oder -4-pyridyl, 1,2,3,4-Tetrahydro-1-, -2-, -3-, -4-, -5-oder -6-pyridyl, 1,2,3,6-Tetrahydro-1-, -2-, -3-, -4-, -5-oder -6-pyridyl, 1-, 2-, 3-, oder 4-Piperidinyl, 2-, 3-oder 4-Morpholinyl, Tetrahydro-2-, -3-oder -4-pyranyl, 1,4-Dioxanyl, 1,3-Dioxan-2-, -4-oder -5-yl, Hexahydro-1-, -3-oder -4-pyridazinyl, Hexahydro-1-, -2-, -4-oder -5-pyrimidyl, 1-, 2-oder 3-Piperazinyl, 1,2,3,4-Tetrahydro-1-, -2-, -3-, -4-, -5-, -6-, -7-, oder -8-chinolyl, 1,2,3,4-Tetrahydro-1-, -2-, -3-, -4-, -5-, -6-, -7-oder -8-isochinolyl.

Die heterocyclischen Reste können auch wie angegeben substituiert sein. Het kann also bevorzugt auch bedeuten: 2-Amino-4-thiazolyl, 4-Carboxy-2-thiazolyl, 4-Carbamoyl-2-thiazolyl, 4-(2-Aminoethyl)-2-thiazolyl, 2-Amino-5,6-dimethyl-3-pyrazinyl, 4-Carbamoylpiperidino, ferner z. B. 3-, 4-oder 5-Methyl-2-furyl, 2-, 4-oder 5-Methyl-3-furyl, 2,4-Dimethyl-3-furyl, 5-Nitro-2-furyl, 5-Styryl-2-furyl, 3-, 4-oder 5-Methyl-2-thienyl, 2-, 4-oder 5-Methyl-3-thienyl, 3-Methyl-5-tert.-butyl-2-thienyl, 5-Chlor-2-thienyl, 5-Phenyl-2-oder -3-thienyl, 1-, 3-, 4-oder 5-Methyl-2-pyrryl, 1-Methyl-4-oder -5-nitro-2-pyrryl, 3,5-Dimethyl-4-ethyl-2-pyrryl, 4-Methyl-5-pyrazolyl, 4-oder 5-Methyl-2-thiazolyl, 2-oder 5-Methyl-4-thiazolyl, 2-oder 4-Methyl-5-thiazolyl, 2,4-Dimethyl-5-thiazolyl, 3-, 4-, 5-oder 6-Methyl-2-pyridyl, 2-, 4-, 5-oder 6-Methyl-3-pyridyl, 2-oder 3-Methyl-4-pyridyl, 3-, 4-, 5-oder 6-Chlor-2-pyridyl, 2-, 4-, 5-oder 6-Chlor-3-pyridyl, 2-oder 3-Chlor-4-pyridyl, 2,6-Dichlorpyridyl, 2-Hydroxy-3-, -4-, -5-oder -6-pyridyl (= 1H-2-Pyridon-3-, -4-, -5-oder -6-yl), 5-Phenyl-1H-2-pyridon-3-yl, 5-p-Methoxyphenyl-1H-2-pyridon-3-yl, 2-Methyl-3-hydroxy-4-hydroxymethyl-5-pyridyl, 2-Hydroxy-4-amino-6-methyl-3-pyridyl, 3-N'-Methylureido-1H-4-pyridon-5-yl, 5-oder 6-Methyl-4-pyrimidyl, 2,6-Dihydroxy-4-pyrimidyl, 5-Chlor-2-methyl-4-pyrimidyl, 2-Methyl-4-amino-5-pyrimidyl, 3-Methyl-2-benzofuryl, 2-Ethyl-3-benzofuryl, 7-Methyl-2-benzothienyl, 1-, 2-, 4-, 5-, 6-oder 7-Methyl-3-indolyl, 1-Methyl-5-oder -6-benzimidazolyl, 1-Ethyl-5-oder -6-benzimidazolyl, 3-, 4-, 5-, 6-, 7-oder 8-Hydroxy-2-chinolyl.

$R^1$ bedeutet vorzugsweise A, insbesondere Methyl, Ethyl, Propyl, Isopropyl, Butyl, Isobutyl oder tert.-Butyl, weiterhin vorzugsweise Cyclopropyl, Cyclopentyl, Cycloheyxl, Phenyl, Benzyl oder Morpholino.

$R^2$ und $R^4$ bedeuten vorzugsweise H oder Methyl, ferner Ethyl, Propyl, Isopropyl, Butyl oder Isobutyl.

$R^3$ bedeutet vorzugweise Cyclohexylmethyl, ferner bevorzugt A, insbesondere Methyl, Ethyl, Propyl, Isopropyl, Butyl, Isobutyl, sek.-Butyl, Pentyl, Isopentyl (3-Methylbutyl) oder 2-Methylbutyl, Phenyl, Benzyl, p-Chlorbenzyl, 2-Cyclohexylethyl, Bicyclo[2,2,1]heptyl-2-methyl oder 6,6-Dimethylbicyclo[3,1,1]heptyl-2-methyl.

$R^5$ ist vorzugsweise H; A, insbesondere Methyl, Ethyl, Isopropyl, Isobutyl, sek.-Butyl, Isopentyl; Ar, insbesondere Phenyl, Cycloalkyl, insbesondere Cyclohexyl.

$R^6$ ist vorzugsweise A, insbesondere Methyl, Ethyl, Propyl, Isopropyl, Butyl, Isobutyl oder sek.-Butyl.

$R^7$ ist vorzugsweise H, Methyl, Ethyl, Isobutyl oder sek.-Butyl, ferner bevorzugt Propyl, Butyl, Cyclohexyl, Cyclohexylmethyl, Phenyl oder Benzyl, weithin bevorzugt Het-alkyl, insbesondere Het-methyl oder 2-Het-ethyl, im einzelnen bevorzugt 5-Tetrazolylmethyl, 2-, 3-oder 4-Pyridylmethyl, 2-(2-, 2-(3-oder 2-(4-Pyridyl)-ethyl, 2-Hydroxy-4,6-dimethyl-3-pyridylmethyl, 2-Methyl-4-amino-5-pyrimidylmethyl oder 2-Amino-5,6-dimethyl-3-pyrazinylmethyl.

$R^8$ ist vorzugsweise H, ferner Methyl, Ethyl oder Phenyl.

L ist vorzugsweise CH.

m, p und r sind vorzugsweise 0, 1 oder 2; n ist vorzugsweise 1; s ist vorzugsweise 1.

X bedeutet vorzugsweise H, POA, Alkoxycarbonyl wie ETOC, IPOC oder BOC, CBZ, Alkanoyl wie Acetyl, Propionyl, Butyryl oder Isobutyryl, Cycloalkylcarbonyl wie Cyclopentylcarbonyl oder Cyclohexylcarbonyl, Aroyl wie Benzoyl, Arylalkanoyl wie Phenylacetyl, 2-oder 3-Phenylpropionyl, 4-Phenylbutyryl, 2-Benzyl-3-phenylpropionyl, PBB, 2-(2-Phenylethyl)-4-phenylbutyryl, 2-(1-Naphthylmethyl)-4-phenylbutyryl, 2-oder 3-o-, -m-oder oder -p-Fluorphenylpropionyl, 2-oder 3-o-, -m-oder -p-Chlorphenylpropionyl, Cycloalkylalkanoyl wie Cyclohexylacetyl, 2-oder 3-Cyclohexylpropionyl, N-Alkylcarbamoyl wie ETNC oder IPNC, MC. Besonders bevorzugte Reste X sind BOC und MC, weiterhin ETOC, IPOC, ETNC, IPNC und PBB, ferner H, POA, 4-Phenylbutyryl, 2-Benzyl-3-phenylpropionyl, 2-(2-Phenylethyl)-4-phenylbutyryl, 2-(1-Naphthylmethyl)-4-phenylbutyryl und CBZ.

Z bedeutet vorzugsweise 2, aber auch 1, weiterhin 3 oder 4 peptidartig miteinander verbundene Aminosäurereste, insbesondere eine der Gruppen His, Phe-His oder Phe-Gly, ferner bevorzugt die Gruppen Abu, Ada, Asn, Bia, Cal, Gln, N-(im)-Methyl-His, Leu, $\alpha$Nal, $\beta$Nal, Nle, Phe, Trp, Tyr, Abu-His, Ada-His, Ala-His, Ala-Phe, Arg-His, Asn-His, Bia-His, Cal-His, Dab-His, Glu-His, His-His, Hph-His, Ile-His, Leu-His, tert.-Leu-His, Lys-His, Met-His, $\alpha$Nal-His, $\beta$Nal-His, Nbg-His, Nle-His, (N-Me-His)-His, (N-Me-Phe)-His, Orn-His, Phe-Abu, Phe-Ada, Phe-Ala, Phe-Arg, Phe-Asn, Phe-Bia, Phe-Cal, Phe-Dab, Phe-Gln, Phe-Glu, Phe-(N-im-Methyl-His), Phe-Ile, Phe-Leu, Phe-tert.-Leu, Phe-Lys, Phe-Met, Phe-$\alpha$-Nal, Phe-$\beta$Nal, Phe-Nbg, Phe-Nle, Phe-(N-Me-His), Phe-(N-Me-Phe), Phe-Orn, Phe-Phe, Phe-Pro, Phe-(2-Pyr), Phe-(3-Pyr), Phe-(4-Pyr), Phe-Ser, Phe-Thr, Phe-Tic, Phe-Trp, Phe-Tyr, Phe-Val, Pro-His, Ser-His, Thr-His, Tic-His, Trp-His, Tyr-His, Val-His, ferner Ada-Phe-His, Pro-Ala-His, Pro-Ala-Phe, Pro-Phe-Ala, Pro-Phe-His, Pro-Phe-Phe, His-Pro-Ala-His, His-Pro-Ala-Phe, His-Pro-Phe-Ala, His-Pro-Phe-Phe, weiterhin Pro-Abu-His, Pro-Ada-His, Pro-Arg-His, Pro-Asn-His, Pro-Bia-His, Pro-Dab-His, Pro-Glu-His, Pro-His-His, Pro-Ile-His, Pro-Leu-His, Pro-tert.-Leu-His, Pro-Lys-His, Pro-Met-His, Pro-Nbg-His, Pro-Nle-His, Pro-(N-Me-His)-His, Pro-(N-Me-Phe)-His, Pro-Orn-His, Pro-Phe-Abu, Pro-Phe-Ada, Pro-Phe-Arg, Pro-Phe-Asn, Pro-Phe-Bia, Pro-Phe-Dab, Pro-Phe-Gln, Pro-Phe-Glu, Pro-Phe-(N-im-Methyl-His), Pro-Phe-Ile, Pro-Phe-Leu, Pro-Phe-tert.-Leu, Pro-Phe-Lys, Pro-Phe-Met, Pro-Phe-Nbg, Pro-Phe-Nle, Pro-Phe-(N-Me-His), Pro-Phe-(N-Me-Phe), Pro-Phe-Orn, Pro-Phe-Pro, Pro-Phe-Ser, Pro-Phe-Thr, Pro-Phe-Tic, Pro-Phe-Trp, Pro-Phe-Tyr, Pro-Phe-Val, Pro-Pro-His, Pro-Ser-His, Pro-Thr-His, Pro-Tic-His, Pro-Trp-His, Pro-Tyr-His, Pro-Val-His, His-Pro-Abu-His, His-Pro-Ada-His, His-Pro-Arg-His, His-Pro-Asn-His, His-Pro-Bia-His, His-Pro-Dab-His, His-Pro-Glu-His, His-Pro-His-His, His-Pro-Ile-His, His-Pro-Leu-His, His-Pro-tert.-Leu-His, His-Pro-Lys-His, His-Pro-Met-His, His-Pro-Nbg-His, His-Pro-Nle-His, His-Pro-(N-Me-His)-His, His-Pro-(N-Me-Phe)-His, His-Pro-Orn-His, His-Pro-Phe-Abu, His-Pro-Phe-Ada, His-Pro-Phe-Arg, His-Pro-Phe-Asn, His-Pro-Phe-Bia, His-Pro-Phe-Dab, His-Pro-Phe-Gln, His-Pro-Phe-Glu, His-Pro-Phe-His, His-Pro-Phe(N-im-Methyl-His), His-Pro-Phe-Ile, His-Pro-Phe-Leu, His-Pro-Phe-tert.-Leu, His-Pro-Phe-Lys, His-Pro-Phe-Met, His-Pro-Phe-Nbg, His-Pro-Phe-Nle, His-Pro-Phe-(N-Me-His), His-Pro-Phe-(N-Me-Phe), His-Pro-Phe-Orn, His-Pro-Phe-Pro, His-Pro-Phe-Ser, His-Pro-Phe-Thr, His-Pro-Phe-Tic, His-Pro-Phe-Trp, His-Pro-Phe-Tyr, His-Pro-Phe-Val, His-Pro-Pro-His, His-Pro-Ser-His, His-Pro-Thr-His, His-Pro-Tic-His, His-Pro-Trp-His, His-Pro-Tyr-His, His-Pro-Val-His.

E bedeutet vorzugsweise -CONH-, ferner bevorzugt -COO-, weiterhin bevorzugt -CSNH-oder -$SO_2$-.

Y ist vorzugsweise $R^5$, -CHA-COOA oder -CHA-$CONR^7R^8$, E-Y auch vorzugsweise Morpholinocarbonyl.

Die Gruppe -$NR^2$-$CHR^3$-CHOH-$(CH_2)_n$-$NR^4$-bedeutet vorzugsweise -NH-$CHR^3$-CHOH-$CH_2$-NH-, insbesondere -NH-CH(Cyclohexylmethyl)-CHOH-$CH_2$-NH-, ferner -NH-CH($CH_2CH_2$-Cyclohexyl)-CHOH-$CH_2$-NH-, -NH-CH(Isobutyl)-CHOH-$CH_2$-NH-oder -NH-CH(Benzyl)-CHOH-$CH_2$-NH-. Diese Gruppe besitzt mindestens ein chirales Zentrum. Die Verbindungen der Formel I können daher in verschiedenen - optisch-inaktiven oder optisch-aktiven - Formen vorkommen. Die Formel I umschließt alle diese Formen. Für n = 1 sind die 2S-Hydroxy-4S-amino-Enantiomeren, für n = 2 entsprechend die 3S-Hydroxy-4S-amino-Enantiomeren bevorzugt.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Id ausgedrückt werden, die der Formel I entsprechen, worin jedoch

in Ia X H, ETOC, IPOC, BOC, POA, CBZ, ETNC, IPNC, MC, N,N-Dimethylcarbamoyl, N,N-Dimethylcarbamoyl, Acetyl, Propionyl, Isovaleryl, 2-Phenylbutyryl, 2-Benzyl-3-phenylpropionyl, PBB, 2-(2-Phenylethyl)-4-phenylbutyryl, 2-(1-Naphthylmethyl)-4-phenylbutyryl, Benzoyl, Ethylsulfonyl, Isopropylsulfonyl oder Morpholinosulfonyl,

Z His; Cal-His, Hph-His, $\alpha$Nal-His, $\beta$Nal-His, Phe-Abu, Phe-Gly, Phe-His, Phe-N(im)-Methyl-His, Phe-Leu, Phe-Met, Phe-Nle, Phe-Phe, Phe-Pyr oder Phe-Trp,

R$^2$ und R$^4$ H,

R$^3$ Butyl, Isobutyl, Cyclohexylmethyl, 2-Cyclohexylethyl oder Benzyl und

n 1 bedeuten;

in Ib X H, ETOC, IPOC, BOC, ETNC, IPNC, MC oder PBB,

Z His, Cal-His, Hph-His, $\alpha$-Nal-His, $\beta$-Nal-His, Trp-His, Tyr-His, Phe-Abu, Phe-Gly, Phe-His, Phe-N-(im)-Methyl-His, Phe-Leu, Phe-Met, Phe-Nle, Phe-Phe, Phe-Pyr oder Phe-Trp,

R$^2$ und R$^4$ H,

R$^3$ Cyclohexylmethyl und

n 1 bedeuten;

in Ic X H, IPOC, BOC oder MC,

Z Phe-Gly oder Phe-His,

R$^2$ und R$^4$ H,

R$^3$ Cyclohexylmethyl oder Benzyl und

n 1 bedeuten;

in Id X BOC,

Z Phe-His,

R$^2$ und R$^4$ H,

R$^3$ Cyclohexylmethyl und

n 1 bedeuten.

Ferner sind bevorzugt Verbindungen der Formeln I' sowie Ia' bis Id', die den Formeln I sowie Ia bis Id entsprechen, worin jedoch

E -CONH-,

Y A, Phenyl, Benzyl, Cyclohexyl, CHA-COOR$^6$ oder CHA-CONR$^7$R$^8$,

R$^6$ Alkyl mit 1-4 C-Atomen,

R$^7$ H,

R$^8$ H oder CH$_2$Het,

Het 3-Amino-5,6-dimethyl-pyrazin-2-yl, 5-Tetrazolyl, Pyridyl, Pyridylmethyl, 2-Hydroxy-4,6-dimethyl-3-pyridyl oder 2-Methyl-4-amino-5-pyrimidyl und

A Alkyl mit 1-5 C-Atomen, vorzugsweise Isopropyl, Isobutyl, sek.-Butyl oder Isopentyl bedeuten.

Ferner sind bevorzugt Verbindungen der Formeln I'' sowie Ia'' bis Id'', die den Formeln I sowie Ia bis Id entsprechen, worin jedoch

E -COO-und

Y Alkyl mit 1-5 C-Atomen, Benzyl oder Cyclohexyl bedeuten.

Ferner sind bevorzugt Verbindungen der Formeln I''' sowie Ia''' bis Id''', die den Formeln I sowie Ia bis Id entsprechen, worin jedoch

E-Y Alkylsulfonyl mit 1-5 C-Atomen, Phenylsulfonyl, Benzylsulfonyl, Cyclohexylsulfonyl, Aminosulfonyl oder Morpholinosulfonyl bedeuten.

Weiterhin sind bevorzugt Verbindungen der Formeln I, Ia bis Id, I', Ia' bis Id', I'', Ia'' bis Id'', I''' sowie Ia''' bis Id''', worin jedoch eine oder mehrere der nachstehenden Voraussetzungen zutreffen, worin nämlich

(a) X von BOC verschieden ist;

(b) X ETOC, IPOC, POA, CBZ, ETNC, IPNC, MC, N,N-Dimethylcarbamoyl, N,N-Diethylcarbamoyl, Acetyl, Propionyl, Isovaleryl, 2-Phenylbutyryl, 2-Benzyl-3-phenylpropionyl, PBB, 2-(2-Phenylethyl)-4-phenylbutyryl, 2-(1-Naphthylmethyl)-4-phenylbutyryl, Benzoyl, Ethylsulfonyl, Isopropylsulfonyl oder Morpholinosulfonyl bedeutet;

(c) X IPOC bedeutet;

(d) X MC bedeutet;

(e) Z von Phe-His verschieden ist;

(f) Z Phe-Gly bedeutet;

(g) R$^3$ von Cyclohexylmethyl verschieden ist;

(h) R$^3$ Benzyl bedeutet;

(i) E -COO-bedeutet;

(j) E -CSNH-bedeutet;

(k) E -SO$_2$-bedeutet;

(l) E -SO$_2$NH-bedeutet;

(m) E -PO(OA)-O-bedeutet;

(n) E-Y -CO-NH-Cyclohexyl bedeutet;

(o) E-Y -CO-NH-(CHR$^5$)$_s$-COOR$^6$ bedeutet;

(p) E-Y -CO-NH-(CHR$^5$)$_s$-CONR$^7$R$^8$ bedeutet;

(q) E-Y Pyrrolidinocarbonyl, Piperidinocarbonyl, Morpholinocarbonyl. Pyrrolidinosulfonyl, Piperidinosulfonyl oder Morpholinosulfonyl bedeutet.

Gegenstand der Erfindung ist ferner ein Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt

oder daß man eine Verbindung, die der Formel I entspricht, aber an Stelle von H-Atomen einer oder mehrere zusätzliche C-C-und/oder C-N-und/oder C-O-Bindungen und/oder O-Atome enthält, reduziert oder daß man eine Carbonsäure der Formel II

X-G$^1$-OH    II

worin G$^1$ (a) Z$^1$,

(b) Z

bedeutet

mit einer Aminoverbindung der Formel III

H-G$^2$    III

worin G$^2$ (a) -Z$^2$-NR$^2$-CHR$^3$-CHOH-(CH$_2$)$_n$-NR$^4$-E-Y,

(b) -NR$^2$-CHR$^3$-CHOH-(CH$_2$)$_n$-NR$^4$-E-Y und

Z$^1$ + Z$^2$ zusammen Z bedeuten

umsetzt,

und daß man gegebenenfalls in einer Verbindung der Formel I eine funktionell abgewandelte Amino und/oder Hydroxygruppe durch Behandeln mit solvolysierenden oder hydrogenolysierenden Mitteln in Freiheit setzt und/oder einen Rest Y durch Behandeln mit veresternden, solvolysierenden, acylierenden oder amidierenden Mitteln in einen anderen Rest Y umwandelt und/oder eine Verbindung der Formel I durch Behandeln mit einer Säure in eines ihrer Salze überführt.

Die Verbindungen der Formel I und auch die Ausgangsstoffe zu ihrer Herstellung werden im übrigen nach an sich bekannten Methoden hergestellt, wie sie in der Literatur (z. B. in den Standardwerken wie Houben-Weyl, Methoden der organischen Chemie, Georg-Thieme-Verlag, Stuttgart; vgl. ferner EP-A-45665, EP-A-77028, EP-A-77029, EP-A-81783 und die übrigen oben angegebenen Druckschriften) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofert weiter zu den Verbindungen der Formel I umsetzt.

Vorzugsweise werden die Verbindungen der Formel I erhalten, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die an Stelle einer oder mehrerer freier Amino-und/oder Hydroxygruppen entsprechende geschützte Amino-und/oder Hydroxygruppe enthalten, vorzugsweise solche, die an Stelle eines H-Atoms, das mit einem N-Atom verbunden ist, eine Aminoschutzgruppe tragen, z. B. solche, die der Formel I entsprechen, aber an Stelle einer His-Gruppe eine N(im)-$R^9$-His-Gruppe (worin $R^9$ eine Aminoschutzgruppe bedeutet, z. B. BOM oder DNP) enthalten.

Ferner sind Ausgangsstoffe bevorzugt, die an Stelle des H-Atoms einer Hydroxygruppe eine Hydroxyschutzgruppe tragen, z. B. solche der Formel X-Z-$NR^2$-$CHR^3$-CHO$R^{10}$-$CH_{2n}$-$NR^4$-E-Y, worin $R^{10}$ eine Hydroxyschutzgruppe bedeutet.

Es können auch mehrere - gleiche oder verschiedene -geschützte Amino-und/oder Hydroxygruppe im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls duchgeführt worden ist. Typisch für solche Gruppen sind insbesondere unsubstituierte oder substituierte Acyl-, Aryl-(z. B. DNP), Aralkoxymethyl-(z. B. BOM) oder Aralkylgruppen (z. B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl-und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-Jodethoxycarbonyl; Aralkyloxycarbonyl wie CBZ ("Carbobenzoxy"), 4-Methoxybenzyloxycarbonyl, FMOC. Bevorzugte Aminoschutzgruppen sind DNP und BOM, ferner CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-Aralkyl-oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäure-und Peptidsynthese hergestellt werden, wie sie z. B. in den genannten Standardwerken und Patentanmeldungen beschrieben sind.

Das In-Freiheit-Setzen der Verbindungen der Formel I aus ihren funktionellen Derivaten gelingt - je nach der benutzten Schutzgruppe - z. B. mit starken Säuren, zweckmäßig mit Trifluoressigsäure oder Perchlorsäure, aber auch mit anderen starken anorganischen Säuren wie Salzsäure oder Schwefelsuaure, starken organischen Carbonsäuren wie Trichloressigsäure oder Sulfonsäuren wie Benzol-oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie Dimethylformamid (DMF), halogenierte Kohlenwasserstoffe wie Dichlormethan, ferner auch Alkohole iwe Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eine weiteren Lösungsmittels verwendet, Perchlorsäure in Form eines Gemisches aus Essigsäure und 70 %iger Perchlorsäure im Verhältnis 9 : 1. Die Reaktionstemperaturen für die Spaltung liegen zweckmäßig zwischen etwa 0 und etwa 50°, vorzugsweise arbeitet man zwischen 15 und 30° (Raumtemperatur).

Die BOC-Gruppe kann z. B. bevorzugt mit 40 %iger Trifluoressigsäure in Methylenchlorid oder mit etwa 3 bis 5 n HCl in Dioxan bei 15-30° abgespalten werden, die FMOC-Gruppe mit einer etwa 5-bis 20%igen Lösung von Dimethylamin, Diethylamin oder Piperidin in DMF bei 15-30°. Eine Abspaltung der DNP-Gruppe gelingt z. B. auch mit einer etwa 3-bis 10%igen Lösung von 2-Mercaptoethanol in DMF/Wasser bei 15-30°.

Hydrogenolytisch entfernbare Schutzgruppen (z. B. BOM, CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5-bis 10 %igem Pd-C in Methanol bei 20-30°.

Die Verbindungen der Formel I sind auch erhältlich durch Reduktion entsprechender Verbindungen, die an Stelle von H-Atomen eine oder mehrere zusätzliche C-C-und/oder C-N-und/oder C-O-Bindungen und/oder O-Atome enthalten.

So sind z. B. Aminoverbindungen der Formel I, die einen Substituenten Ar = Aminophenyl enthalten, durch Reduktion der entsprechenden Nitroverbindungen erhältlich, z. B. durch katalytische Hydrierung unter den vorstehend für die Hydrogenolyse genannten Bedingungen.

Verbindungen der Formel I können auch durch direkte Peptidsynthese aus einer Carbonsäure-(Formel II) und einer Aminkomponente (Formel III) erhalten werden. Als Carbonsäurekomponenten eignen sich z. B. solche der Teilformel X-Z-OH, als Aminkomponenten solche der Teilformel H-NR$^2$-CHR$^3$-CHOH-(CH$_2$)$_n$-NR$^4$-E-Y. Die Peptidbindung kann aber auch innerhalb der Gruppe Z geknüpft werden; dabei wird eine Carbonsäure der Formel X-Z$^1$-OH mit einer Aminoverbindung der Formel H-Z$^2$-NR$^2$-CHR$^3$-CHOH-(CH$_2$)$_n$-NR$^4$-E-Y umgesetzt, wobei Z$^1$ + Z$^2$ = Z ist. Dabei arbeitet man zweckmäßig nach üblichen Methoden der Peptid-Synthese, wie sie z. B. in Houben-Weyl, 1.c., Band 15/II, Seiten 1 bis 806 (1974) beschrieben sind.

Die Reaktion gelingt vorzugsweise in Gegenwart eines Dehydratisierungsmittels, z. B. eines Carbodiimids wie DDCI oder Dimethylaminopropylethyl-carbodiimid, ferner Propanphosphonsäureanhydrid (vgl. Angew.Chem. 92, 129 (1980)), Diphenylphosphorylazid oder 2-Ethoxy-N-ethoxy-carbonyl-1,2-dihydrochinolin, in einem inerten Lösungsmittel, z. B. einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, bei Temperaturen zwischen etwa -10 und 40, vorzugsweise zwischen 0 und 30°.

An Stelle von II bzw. III können auch geeignete reaktionsfähige Derivate dieser Stoffe in die Reaktion eingesetzt werden, z. B. solche, in denen reaktive Gruppen intermediär durch Schutzgruppen blockiert sind. Die Aminosäurederivate III können z. B. in Form ihrer aktivierten Ester verwendet werden, die zweckmäßig in situ gebildet werden, z. B. durch Zusatz von HOBt oder N-Hydroxysuccinimid.

Die Ausgangsttoffe der Formeln II und III sind großenteils bekannt. Sofern sie nicht bekannt sind, können sie nach bekannten Methoden, z. B. den oben angegebenen Methoden der Peptidsynthese und der Abspaltung von Schutzgruppen, hergestellt werden.

Gewünschtenfalls kann in einer Verbindung der Formel I eine funktionell abgewandelte Amino-und/oder Hydroxygruppe durch Solvolyse oder Hydrogenolyse nach einer der oben beschriebenen Methoden in Freiheit gesetzt werden.

So kann insbesondere eine Verbindung der Formel I, worin X von H verschieden ist, in eine Verbindung der Formel I (X = H) umgewandelt werden, zweckmäßig durch Hydrogenolyse, falls X = CBZ ist, sonst durch selektive Solvolyse. Falls X = BOC ist, kann man z. B. mit HCl in Dioxan bei Raumtemperatur die BOC-Gruppe abspalten.

Weiterhin ist es möglich, einen Rest Y durch Behandeln mit veresternden, solvolysierenden, acylierenden oder amidierenden Mitteln in einen anderen Rest Y umzuwandeln. So kann man eine Säure verestern, z. B. mit Hilfe eines Alkohols der Formel A-OH oder eines Diazoalkans, z. B. Diazomethan, oder man kann einen Ester zur entsprechenden Säure verseifen, z. B. mit wäßrig-dioxanischer Natronlauge bei Raumtemperatur. Ferner kann man z. B. einen Ester durch Behandeln mit Ammoniak oder mit einem Amin der Formel A-NH$_2$ oder A$_2$NH in das entsprechende Amid überführen.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein-oder mehrbasige Carbon-, Sulfon-oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-oder 3-Phenylpropionsäure, Citronensäure, Gluconsäure, Ascorbinsäure, Nicotinsäure, Isonicotinsäure, Methan-oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z. B. Pikrate, können zur Isolierung und/oder Aufreinigung der Verbindungen der Formel I verwendet werden.

Die neuen Verbindungen der Formel I und ihre physiologisch unbedenklichen Salze können zur Herstellung pharmazeutischer Präparate verwendet werden, indem man sie zusammen mit mindestens einem Träger-oder Hilfsstoff und, falls erwünscht, zusammen mit einem oder mehreren weiteren Wirkstoff(en) in eine geeignete Dosierungsform bringt. Die so erhaltenden Zubereitungen können als Arzneimittel in der Human-oder Veterinärmedizin eingesetzt werden. Als Trägersubstanzen kommen organische oder anorganische Stoffe in Frage, die sich für die enterale (z. B. orale oder rektale) oder parenterale Applikation oder für eine Applikation in Form eines Inhalationssprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z. B. Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol, Inhalationslösungen können mit Hilfe üblicher Inhalatorenverabfolgt werden. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Konservierungs-, Stabiliserungs-und/oder Netzmittel, Emulgatoren, Salze zur Beeinflussung des osmotischen Druckes, Puffersubstanzen, Farb-und/oder Aromastoffe enthalten. Sie können, falls erwünscht, auch einen oder mehrere weitere Wirkstoffe enthalten, z. B. ein oder mehrere Vitamine.

Die erfindungsgemäßen Substanzen werden in der Regel in Analogie zu anderen bekannten, im Handel befindlichen Peptiden, insbesondere aber in Analogie zu den in der EP-A-77028 beschriebenen Verbindungen verabreicht, vorzugsweise in Dosierungen zwischen etwa 100 mg und 30 g, insbesondere zwischen 500 mg und 5 g pro Dosierungseinheit. Die tägliche Dosierung liegt vorzugsweise zwischen etwa 2 und 600 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt -und weg, von der Ausscheidungsgeschwindigkeit, Arzneistoffkombination und Schwere der jeweiligen Erkrankung, welcher die Therapie gilt. Die parenterale Applikation ist bevorzugt.

Vor-und nachstehend sind alle Temperaturen in °C angegeben. In den nachfolgenden Beispielen bedeutet "übliche Aufarbeitung": Man gibt, falls erforderlich, Wasser hinzu, neutralisiert, extrahiert mit Ether oder Dichlormethan, trennt ab, trocknet die organische Phase über Natriumsulfat, filtriert, dampft ein und reinigt durch Chromatographie an Kieselgel und/oder Kristallisation.

Beispiel 1

Ein Gemisch von 1 g N-Isopentyl-N'-[2S-hydroxy-3S-(N-tert.-butoxycarbonyl-L-phenylalanyl-N(im)-(2,4-dinitrophenyl)-L-histidyl-amino)-4-cyclohexyl-butyl]-harnstoff [N-Isopentyl-N'-[2S-hydroxy-3S-(BOC-Phe-(imi-DNP-His)-amino)-4-cyclohexyl-butyl]-harnstoff; erhältlich durch Reaktion von 1-Brom-3S-BOC-amino-4-cyclohexyl-butan-2-on mit $NaN_3$ in DMF bei 0° zu 1-Azido-3S-BOC-amino-4-cyclohexylbutan-2-on (F. 58-59 °), Reduktion mit $NaBH_4$ mit Methanol zu 1-Azido-3S-BOC-amino-4-cyclohexyl-butan-2S-ol (F. 170-171°; daneben entsteht 1-Azido-3S-BOC-amino-4-cyclohexyl-butan-2R-ol, F. 69-71 °, das chromatographisch von dem 2S-Epimeren getrennt wird), Hydrierung an Pd-C in Methanol zu 1-Amino-3S-BOC-amino-4-cyclohexyl-butan-2S-ol (F. 110-111 °; analog; 1-Amino-3S-BOC-amino-4-cyclohexyl-butan-2R-ol, F. 116-117 °), Umsetzung mit Isopentylisocyanat in THF (2 Std. bei 20 °) zu N-Isopentyl-N'-(2S-hydroxy-3S-BOC-amino-4-cyclohexyl-butyl)-harnstoff (F. 160-162 °), Abspaltung der BOC-Gruppe mit 4 n HCl in Dioxan, Reaktion mit BOC-(imi-DNP-His)-OH zu N-Isopentyl-N'-[2S-hydroxy-3S-(BOC-(imi-DNP-His)-amino)-4-cyclohexylbutyl]-harnstoff, erneute Abspaltung der BOC-Gruppe und Reaktion mit BOC-Phe-OH], 2 g 2-Mercaptoethanol, 20 ml DMF und 20 ml Wasser wird unter Rühren bei 20 ° mit wässeriger $Na_2CO_3$-Lösung auf pH 8 eingestellt und 2 Std. bei 20 ° gerührt. Nach üblicher Aufarbeitung erhält man N-Isopentyl-N'-[2S-hydroxy-3S-(BOC-Phe-His-amino)-4-cyclohexyl-butyl]-harnstoff, F. 132-134 °.

Analog erhält man durch Spaltung der entsprechenden (imi-DNP-His)-Derivate:
N-(2S-Hydroxy-3S-BOC-αNal-His-amino-4-cyclohexyl-butyl)-harnstoff [F. 134°; über N-[2S-Hydroxxy-3S-BOC-(imi-DNP-His)-amino-4-cyclohexyl-butyl]-harnstoff [F. 82° (Zersetzung)] und N-[2S-Hydroxy-3S-BOC-αNal-(imi-DNP-His)-amino-4-cyclohexyl-butyl]-harnstoff (F. 107-108°)].

N-[2S-Hydroxy-3S-(BOC-Phe-His-amino)-4-cyclohexyl-butyl]-harnstoff [F. 156°; durch Reaktion von 2S-tert.-Butyldimethylsilyloxy-3S-BOC-amino-4-cyclohexyl-butylamin mit Trimethylsilyl-isocyanat in THF zu N-(2S-tert.-Butyldimethylsilyloxy-3S-BOC-amino-4-cyclohexyl-butyl)-harnstoff (F. 61°) und weiter über N-[2S-Hydroxy-3S-BOC-(imi-DNP-His)-amino-4-cyclohexyl-butyl]-harnstoff und N-[2S-Hydroxy-3S-BOC-Phe-(imi-DNP-His)-amino-4-cyclohexyl-butyl]-harnstoff] N-Methyl-N'-[2S-hydroxy-3S-(BOC-Phe-His-amino)-4-cyclohexyl-butyl]-harnstoff
N-Methyl-N'-[2S-hydroxy-3S-(BOC-Phe-His-amino)-4-cyclohexyl-butyl]-harnstoff
N-Ethyl-N'-[2S-hydroxy-3S-(BOC-Phe-His-amino)-4-cyclohexyl-butyl]-harnstoff
N-Propyl-N'-[2S-hydroxy-3S-(BOC-Phe-His-amino)-4-cyclohexyl-butyl]-harnstoff
N-Isopropyl-N'-[2S-hydroxy-3S-(PBB-His-amino)-4-cyclohexyl-butyl]-harnstoff
N-Isopropyl-N'-[2S-hydroxy-3S-(POA-His-amino)-4-cyclohexyl-butyl]-harnstoff
N-Isopropyl-N'-[2S-hydroxy-3S-(PBB-Phe-His-amino)-4-cyclohexyl-butyl]-harnstoff
N-Isopropyl-N'-[2S-hydroxy-3S-(ETOC-Phe-His-amino)-4-cyclohexyl-butyl]-harnstoff
N-Isopropyl-N'-[2S-hydroxy-3S-(IPOC-Phe-His-amino)-4-cyclohexyl-butyl]-harnstoff, F. 206-208°
N-Isopropyl-N'-[2S-hydroxy-3S-(BOC-Phe-His-amino)-4-cyclohexyl-butyl]-harnstoff
N-Isopropyl-N'-[2S-hydroxy-3S-(CBZ-Phe-His-amino)-4-cyclohexyl-butyl]-harnstoff
N-Isopropyl-N'-[2S-hydroxy-3S-(ETNC-Phe-His-amino)-4-cyclohexyl-butyl]-harnstoff
N-Isopropyl-N'-[2S-hydroxy-3S-(IPNC-Phe-His-amino)-4-cyclohexyl-butyl]-harnstoff
N-Isopropyl-N'-[2S-hydroxy-3S-(N,N-dimethylcarbamoyl-Phe-His-amino)-4-cyclohexyl-butyl]-harnstoff
N-Isopropyl-N'-[2S-hydroxy-3S-(N,N-diethylcarbamoyl-Phe-His-amino)-4-cyclohexyl-butyl]-harnstoff
N-Isopropyl-N'-[2S-hydroxy-3S-(MC-Phe-His-amino)-4-cyclohexyl-butyl]-harnstoff
N-Isopropyl-N'-[2S-hydroxy-3S-(acetyl-Phe-His-amino)-4-cyclohexyl-butyl]-harnstoff
N-Isopropyl-N'-[2S-hydroxy-3S-(propionyl-Phe-His-amino)-4-cyclohexyl-butyl]-harnstoff
N-Isopropyl-N'-[2S-hydroxy-3S-(isovaleryl-Phe-His-amino)-4-cyclohexyl-butyl]-harnstoff
N-Isopropyl-N'-[2S-hydroxy-3S-(benzoyl-Phe-His-amino)-4-cyclohexyl-butyl]-harnstoff
N-Isopropyl-N'[-2S-hydroxy-3S-(POA-Phe-His-amino)-4-cyclohexyl-butyl]-harnstoff
N-Isopropyl-N'-[2S-hydroxy-3S-(ethylsulfonyl-Phe-His-amino)-4-cyclohexyl-butyl]-harnstoff
N-Isopropyl-N'-[2S-hydroxy-3S-(isopropylsulfonyl-Phe-His-amino)-4-cyclohexyl-butyl]-harnstoff
N-Isopopyl-N'-[2S-hydroxy-3S-(morpholinosulfonyl-Phe-His-amino)-4-cyclohexyl-butyl]-harnstoff
N-Isobutyl-N'-[2S-hydroxy-3S-(BOC-Phe-His-amino)-4-cyclohexyl-butyl]-harnstoff
N-sek.-Butyl-N'-[2S-hydroxy-3S-(BOC-Phe-His-amino)-4-cyclohexyl-butyl]-harnstoff, F. 199°, (Zersetzung)
N-(2-Methyl-butyl)-N'-[2S-hydroxy-3S-(BOC-Phe-His-amino)-4-cyclohexyl-butyl]-harnstoff
N-Isopentyl-N'-[2S-hydroxy-3S-(PBB-His-amino)-4-cyclohexyl-butyl]-harnstoff
N-Isopentyl-N'-[2S-hydroxy-3S-(POA-His-amino)-4-cyclohexyl-butyl]-harnstoff
N-Isopentyl-N'-[2S-hydroxy-3S-(PBB-Phe-His-amino)-4-cyclohexyl-butyl]-harnstoff
N-Isopentyl-N'-[2S-hydroxy-3S-(ETOC-Phe-His-amino)-4-cyclohexyl-butyl]-harnstoff
N-Isopentyl-N'-[2S-hydroxy-3S-(IPOC-Phe-His-amino)-4-cyclohexyl-butyl]-harnstoff
N-Isopentyl-N'-[2S-hydroxy-3S-(CBZ-Phe-His-amino)-4-cyclohexyl-butyl]-harnstoff
N-Isopentyl-N'-[2S-hydroxy-3S-(ETNC-Phe-His-amino)-4-cyclohexyl-butyl]-harnstoff
N-Isopentyl-N'-[2S-hydroxy-3S-(IPNC-Phe-His-amino)-4-cyclohexyl-butyl]-harnstoff
N-Isopentyl-N'-[2S-hydroxy-3S-(N,N-dimethylcarbamoyl-Phe-His-amino)-4-cyclohexyl-butyl]-harnstoff
N-Isopentyl-N'-[2S-hydroxy-3S-(N,N-diethylcarbamoyl-Phe-His-amino)-4-cyclohexyl-butyl]-harnstoff
N-Isopentyl-N'-[2S-hydroxy-3S-(MC-Phe-His-amino)-4-cyclohexyl-butyl]-harnstoff

N-Isopentyl-N'-[2S-hydroxy-3S-(acetyl-Phe-His-amino)-4-cyclohexyl-butyl]-harnstoff

N-Isopentyl-N'-[2S-hydroxy-3S-(propionyl-Phe-His-amino)-4-cyclohexyl-butyl]-harnstoff

N-Isopentyl-N'-[2S-hydroxy-3S-(isovaleryl-Phe-His-amino)-4-cyclohexyl-butyl]-harnstoff

N-Isopentyl-N'-[2S-hydroxy-3S-(benzoyl-Phe-His-amino)-4-cyclohexyl-butyl]-harnstoff

N-Isopentyl-N'-[2S-hydroxy-3S-(POA-Phe-His-amino)-4-cyclohexyl-butyl]-harnstoff

N-Isopentyl-N'-[2S-hydroxy-3S-(ethylsulfonyl-Phe-His-amino)-4-cyclohexyl-butyl]-harnstoff

N-Isopentyl-N'-[2S-hydroxy-3S-(isopropylsulfonyl-Phe-His-amino)-4-cyclohexyl-butyl]-harnstoff

N-Isopentyl-N'-[2S-hydroxy-3S-(morpholinosulfonyl-Phe-His-amino)-4-cyclohexyl-butyl]-harnstoff

N-Cyclohexyl-N'-[2S-hydroxy-3S-(IPOC-Phe-His-amino)-4-cyclohexyl-butyl]-harnstoff, F. 189° [Zersetzung; über N-Cyclohexyl-N'-[2S-hydroxy-3S-BOC-(imi-DNP-His)-amino-4-cyclohexyl-butyl]-harnstoff [F. 80° (Zersetzung)] und N-Cyclohexyl-N'-[2S-hydroxy-3S-IPOC-Phe-(imi-DNP-His)-amino-4-cyclohexyl-butyl]-harnstoff [F. 182° (Zersetzung)]] N-Cyclohexyl-N'-[2S-hydroxy-3S-(BOC-Phe-His-amino)-4-cyclohexyl-butyl]-harnstoff, F. 146° [Zersetzung; über 2S-tert.-Butyldimethylsilyloxy-3S-BOC-amino-4-cyclohexyl-butylamin (Öl), N-Cyclohexyl-N'-(2S-tert.-butyldimethylsilyloxy-3S-BOC-amino-4-cyclohexyl-butyl)-harnstoff (F. 75-76°), N-Cyclohexyl-N'-[2S-hydroxy-3S-BOC-(imi-DNP-His)-amino-4-cyclohexyl-butyl]-harnstoff [F. 80° (Zersetzung)] und N-Cyclohexyl-N'-[2S-hydroxy-3S-BOC-Phe-(imi-DNP-His)-amino-4-cyclohexyl-butyl]-harnstoff [F. 154° (Zersetzung)]]

N-Phenyl-N'-[2S-hydroxy-3S-(BOC-Phe-His-amino)-4-cyclohexyl-butyl]-harnstoff

N,N-(3-Oxapentamethylen)-N'-[2S-hydroxy-3S-(BOC-Phe-His-amino)-4-cyclohexyl-butyl]-harnstoff

N-[2R-Hydroxy-3S-(BOC-Phe-His-amino)-4-cyclohexyl-butyl]-harnstoff

N-Methyl-N'-[2R-hydroxy-3S-(BOC-Phe-His-amino)-4-cyclohexyl-butyl]-harnstoff

N-Ethyl-N'-[2R-hydroxy-3S-(BOC-Phe-His-amino)-4-cyclohexyl-butyl]-harnstoff

N-Propyl-N'-[2R-hydroxy-3S-(BOC-Phe-His-amino)-4-cyclohexyl-butyl]-harnstoff

N-Isopropyl-N'-[2R-hydroxy-3S-(PBB-His-amino)-4-cyclohexyl-butyl]-harnstoff

N-Isopropyl-N'-[2R-hydroxy-3S-(POA-His-amino)-4-cyclohexyl-butyl]-harnstoff

N-Isopropyl-N'-[2R-hydroxy-3S-(PBB-Phe-His-amino)-4-cyclohexyl-butyl]-harnstoff

N-Isopropyl-N'-[2R-hydroxy-3S-(ETOC-Phe-His-amino)-4-cyclohexyl-butyl]-harnstoff

N-Isopropyl-N'-[2R-hydroxy-3S-(IPOC-Phe-His-amino)-4-cyclohexyl-butyl]-harnstoff

N-Isopropyl-N'-[2R-hydroxy-3S-(BOC-Phe-His-amino)-4-cyclohexyl-butyl]-harnstoff, F. 139°

N-Isopropyl-N'-[2R-hydroxy-3S-(CBZ-Phe-His-amino)-4-cyclohexyl-butyl]-harnstoff

N-Isopropyl-N'-[2R-hydroxy-3S-(ETNC-Phe-His-amino)-4-cyclohexyl-butyl]-harnstoff

N-Isopropyl-N'-[2R-hydroxy-3S-(IPNC-Phe-His-amino)-4-cyclohexyl-butyl]-harnstoff

N-Isopropyl-N'-[2R-hydroxy-3S-(N,N-dimethylcarbamoyl-Phe-His-amino)-4-cyclohexyl-butyl]-harnstoff

N-Isopropyl-N'-[2R-hydroxy-3S-(N,N-diethylcarbamoyl-Phe-His-amino)-4-cyclohexyl-butyl]-harnstoff

N-Isopropyl-N'-[2R-hydroxy-3S-(MC-Phe-His-amino)-4-cyclohexyl-butyl]-harnstoff

N-Isopropyl-N'-[2R-hydroxy-3S-(acetyl-Phe-His-amino)-4-cyclohexyl-butyl]-harnstoff

N-Isopropyl-N'-[2R-hydroxy-3S-(propionyl-Phe-His-amino)-4-cyclohexyl-butyl]-harnstoff

N-Isopropyl-N'-[2R-hydroxy-3S-(isovaleryl-Phe-His-amino)-4-cyclohexyl-butyl]-harnstoff

N-Isopropyl-N'-[2R-hydroxy-3S-(benzoyl-Phe-His-amino)-4-cyclohexyl-butyl]-harnstoff

N-Isopropyl-N'-[2R-hydroxy-3S-(POA-Phe-His-amino)-4-cyclohexyl-butyl]-harnstoff

N-Isopropyl-N'-[2R-hydroxy-3S-(ethylsulfonyl-Phe-His-amino)-4-cyclohexyl-butyl]-harnstoff

N-Isopropyl-N'-[2R-hydroxy-3S-(isopropylsulfonyl-Phe-His-amino)-4-cyclohexyl-butyl]-harnstoff

N-Isopropyl-N'-[2R-hydroxy-3S-(morpholinosulfonyl-Phe-His-amino)-4-cyclohexyl-butyl]-harnstoff

N-Isobutyl-N'-[2R-hydroxy-3S-(BOC-Phe-His-amino)-4-cyclohexyl-butyl]-harnstoff

N-sek.-Butyl-N'-[2R-hydroxy-3S-(BOC-Phe-His-amino)-4-cyclohexyl-butyl]-harnstoff

N-(2-Methyl-butyl)-N'-[2R-hydroxy-3S-(BOC-Phe-His-amino)-4-cyclohexyl-butyl]-harnstoff

N-Isopentyl-N'-[2R-hydroxy-3S-(PBB-His-amino)-4-cyclohexylbutyl]-harnstoff

N-Isopentyl-N'-[2R-hydroxy-3S-(POA-His-amino)-4-cyclohexylbutyl]-harnstoff

N-Isopentyl-N'-[2R-hydroxy-3S-(PBB-Phe-His-amino)-4-cyclohexyl-butyl]-harnstoff

N-Isopentyl-N'-[2R-hydroxy-3S-(ETOC-Phe-His-amino)-4-cyclohexyl-butyl]-harnstoff

N-Isopentyl-N'-[2R-hydroxy-3S-(IPOC-Phe-His-amino)-4-cyclohexyl-butyl]-harnstoff

N-Isopentyl-N'-[2R-hydroxy-3S-(BOC-Phe-His-amino)-4-cyclohexyl-butyl]-harnstoff

N-Isopentyl-N'-[2R-hydroxy-3S-(CBZ-Phe-His-amino)-4-cyclohexyl-butyl]-harnstoff

N-Isopentyl-N'-[2R-hydroxy-3S-(ETNC-Phe-His-amino)-4-cyclohexyl-butyl]-harnstoff

N-Isopentyl-N'-[2R-hydroxy-3S-(IPNC-Phe-His-amino)-4-cyclohexyl-butyl]-harnstoff

N-Isopentyl-N'-[2R-hydroxy-3S-(N,N-dimethylcarbamoyl-Phe-His-amino)-4-cyclohexyl-butyl]-harnstoff

N-Isopentyl-N'-[2R-hydroxy-3S-(N,N-diethylcarbamoyl-Phe-His-amino)-4-cyclohexyl-butyl]-harnstoff

N-Isopentyl-N'-[2R-hydroxy-3S-(MC-Phe-His-amino)-4-cyclohexyl-butyl]-harnstoff

N-Isopentyl-N'-[2R-hydroxy-3S-(acetyl-Phe-His-amino)-4-cyclohexyl-butyl]-harnstoff

N-Isopentyl-N'-[2R-hydroxy-3S-(propionyl-Phe-His-amino)-4-cyclohexyl-butyl]-harnstoff

N-Isopentyl-N'-[2R-hydroxy-3S-(isovaleryl-Phe-His-amino)-4-cyclohexyl-butyl]-harnstoff

N-Isopentyl-N'-[2R-hydroxy-3S-(benzoyl-Phe-His-amino)-4-cyclohexyl-butyl]-harnstoff

N-Isopentyl-N'-[2R-hydroxy-3S-(POA-Phe-His-amino)-4-cyclohexyl-butyl]-harnstoff

N-Isopentyl-N'-[2R-hydroxy-3S-(ethylsulfonyl-Phe-His-amino)-4-cyclohexy-butyl]-harnstoff

N-Isopentyl-N'-[2R-hydroxy-3S-(isopropylsulfonyl-Phe-His-amino)-4-cyclohexyl-butyl]-harnstoff

N-Isopentyl-N'-[2R-hydroxy-3S-(morpholinosulfonyl-Phe-His-amino)-4-cyclohexyl-butyl]-harnstoff

N-Cyclohexyl-N'-[2R-hydroxy-3S-(IPOC-Phe-His-amino)-4-cyclohexyl-butyl]-harnstoff

N-Cyclohexyl-N'-[2R-hydroxy-3S-(BOC-Phe-His-amino)-4-cyclohexyl-butyl]-harnstoff

N-Phenyl-N'-[2R-hydroxy-3S-(BOC-Phe-His-amino)-4-cyclohexyl-butyl]-harnstoff

N,N-(3-Oxapentamethylen)-N'-[2R-hydroxy-3S-(BOC-Phe-His-amino)-4-cyclohexyl-butyl]-harnstoff

N-[2S-Hydroxy-3S-(BOC-Phe-His-amino)-4-cyclohexyl-butyl]-isopropylsulfonamid [F. 117° (Zersetzung); über N-[2S-Hydroxy-3S-BOC-(imi-DNP-His)-amino-4-cyclohexyl-butyl]-isopropylsulfonamid [F. 87° (Zersetzung)] und N-[2S-Hydroxy-3S-BOC-Phe-(imi-DNP-His)-amino-4-cyclohexyl-butyl]-isopropylsulfonamid (F. 130°)].


Beispiel 2

Man löst 1 g N-Isopentyl-N'-[2S-hydroxy-3S-BOC-Phe-(imi-BOM-His)-amino-4-cyclohexylbutyl]-harnstoff [erhältlich durch Reaktion von N-Isopentyl-N'-(2S-hydroxy-3S-amino-4-cyclohexyl-butyl)-harnstoff mit BOC-(imi-BOM-His)-OH zu N-Isopentyl-N'-[2S-hydroxy-3S-BOC-(imi-BOM-His)-amino-4-cyclohexyl-butyl]-harnstoff, Abspaltung der BOC-Gruppe und Reaktion mit BOC-Phe-OH] in 10 ml Methanol, hydriert an 5%ig. Pd-C bei 20° und 1 bar bis zum Stillstand, filtriert, dampft ein und erhält N-Isopentyl-N'-[2S-hydroxy-3S-(BOC-Phe-His-amino)-4-cyclohexyl-butyl]-harnstoff, F. 132-134°.

Analog erhält man durch Hydrogenolyse der entsprechenden imi-BOM-His-Verbindungen:
N-Isopropyl-N'-[2R-hydroxy-3S-(BOC-Phe-His-amino)-4-cyclohexyl-butyl]-harnstoff, F. 139° [über N-Isopropyl-N'-[2R-hydroxy-3S-BOC-(imi-BOM-His)-amino-4-cyclohexyl-butyl]-harnstoff [F. 214° (Zersetzung)] und N-Isopropyl-N'-[2R-hydroxy-3S-BOC-Phe-(imi-BOM-His)-amino-4-cyclohexyl-butyl]-harnstoff (F. 104°)];

N-Isopropyl-N'-[2S-hydroxy-3S-(IPOC-Phe-His-amino)-4-cyclohexyl-butyl]-harnstoff, F. 206-208° [über N-Isopropyl-N'-(2S-tert.-butyldimethylsilyloxy-3S-BOC-amino-4-cyclohexyl-butyl)-harnstoff (F. 131°) und N-Isopropyl-N'-[2S-hydroxy-3S-BOC-(imi-BOM-His)-amino-4-cyclohexylbutyl]-harnstoff [F. 184° (Zersetzung)]];

N-Isopropyl-N'-[2R-hydroxy-3S-(MC-Phe-His-amino)-4-cyclohexyl-butyl]-harnstoff, F 152° [Zersetzung; über N-Isopropyl-N-(2R-hydroxy-3S-amino-4-cyclohexyl-butyl)-harnstoff (Hydrochlorid, F. 84°), N-Isopropyl-N'-[2R-hydroxy-3S-BOC-(imi-BOM-His)-amino-4-cyclohexyl-butyl]-harnstoff [F. 214° (Zersetzung)] und N-Isopropyl-N'-[2R-hydroxy-3S-MC-Phe-(imi-BOM-His)-amino-4-cyclohexyl-butyl]-harnstoff [F. 79° (Zersetzung)];

N-Isopropyl-N'-[2S-hydroxy-3S-(ETOC-Phe-His-amino)-heptyl]-harnstoff

N-Isopropyl-N'-[2S-hydroxy-3S-(IPOC-Phe-His-amino)-heptyl]-harnstoff

N-Isopropyl-N'-[2S-hydroxy-3S-(BOC-Phe-His-amino)-heptyl]-harnstoff

N-Isopropyl-N'-[2S-hydroxy-3S-(ETNC-Phe-His-amino)-heptyl]-harnstoff

N-Isopropyl-N'-[2S-hydroxy-3S-(IPNC-Phe-His-amino)-heptyl]-harnstoff

N-Isopropyl-N'-[2S-hydroxy-3S-(MC-Phe-His-amino)-heptyl]-harnstoff

N-Isopropyl-N'-[2S-hydroxy-3S-(ETOC-Phe-His-amino)-5-methyl-hexyl]-harnstoff

N-Isopropyl-N'-[2S-hydroxy-3S-(IPOC-Phe-His-amino)-5-methyl-hexyl]-harnstoff

N-Isopropyl-N'-[2S-hydroxy-3S-(BOC-Phe-His-amino)-5-methyl-hexyl]-harnstoff

N-Isopropyl-N'-[2S-hydroxy-3S-(ETNC-Phe-His-amino)-5-methyl-hexyl]-harnstoff

N-Isopropyl-N'-[2S-hydroxy-3S-(IPNC-Phe-His-amino)-5-methyl-hexyl]-harnstoff

N-Isopropyl-N'-[2S-hydroxy-3S-(MC-Phe-His-amino)-5-methyl-hexyl]-harnstoff

N-Isopropyl-N'-[2S-hydroxy-3S-(ETOC-Phe-His-amino)-4-phenyl-butyl]-harnstoff

N-Isopropyl-N'-[2S-hydroxy-3S-(IPOC-Phe-His-amino)-4-phenyl-butyl]-harnstoff

N-Isopropyl-N'-[2S-hydroxy-3S-(BOC-Phe-His-amino)-4-phenyl-butyl]-harnstoff, F. 208° (Zersetzung)

N-Isopropyl-N'-[2S-hydroxy-3S-(ETNC-Phe-His-amino)-4-phenyl-butyl]-harnstoff

N-Isopropyl-N'-[2S-hydroxy-3S-(IPNC-Phe-His-amino)-4-phenyl-butyl]-harnstoff

N-Isopropyl-N'-[2S-hydroxy-3S-(MC-Phe-His-amino)-4-phenyl-butyl]-harnstoff

N-Isopentyl-N'-[2S-hydroxy-3S-(ETOC-Phe-His-amino)-heptyl]-harnstoff

N-Isopentyl-N'-[2S-hydroxy-3S-(IPOC-Phe-His-amino)-heptyl]-harnstoff
N-Isopentyl-N'-[2S-hydroxy-3S-(BOC-Phe-His-amino)-heptyl]-harnstoff
N-Isopentyl-N'-[2S-hydroxy-3S-(ETNC-Phe-His-amino)-heptyl]-harnstoff
N-Isopentyl-N'-[2S-hydroxy-3S-(IPNC-Phe-His-amino)-heptyl]-harnstoff
N-Isopentyl-N'-[2S-hydroxy-3S-(MC-Phe-His-amino)-heptyl]-harnstoff
N-Isopentyl-N'-[2S-hydroxy-3S-(ETOC-Phe-His-amino)-5-methyl-hexyl]-harnstoff
N-Isopentyl-N'-[2S-hydroxy-3S-(IPOC-Phe-His-amino)-5-methyl-hexyl]-harnstoff
N-Isopentyl-N'-[2S-hydroxy-3S-(BOC-Phe-His-amino)-5-methyl-hexyl]-harnstoff
N-Isopentyl-N'-[2S-hydroxy-3S-(ETNC-Phe-His-amino)-5-methyl-hexyl]-harnstoff
N-Isopentyl-N'-[2S-hydroxy-3S-(IPNC-Phe-His-amino)-5-methyl-hexyl]-harnstoff
N-Isopentyl-N'-[2S-hydroxy-3S-(MC-Phe-His-amino)-5-methyl-hexyl]-harnstoff
N-Isopentyl-N'-[2S-hydroxy-3S-(ETOC-Phe-His-amino)-4-phenyl-butyl]-harnstoff
N-Isopentyl-N'-[2S-hydroxy-3S-(IPOC-Phe-His-amino)-4-phenyl-butyl]-harnstoff
N-Isopentyl-N'-[2S-hydroxy-3S-(BOC-Phe-His-amino)-4-phenyl-butyl]-harnstoff
N-Isopentyl-N'-[2S-hydroxy-3S-(ETNC-Phe-His-amino)-4-phenyl-butyl]-harnstoff
N-Isopentyl-N'-[2S-hydroxy-3S-(IPNC-Phe-His-amino)-4-phenyl-butyl]-harnstoff
N-Isopentyl-N'-[2S-hydroxy-3S-(MC-Phe-His-amino)-4-phenyl-butyl]-harnstoff
N-Isopropyl-N'-[2S-hydroxy-3S-(BOC-Cal-His-amino)-4-cyclohexyl-butyl]-harnstoff
N-Isopropyl-N'-[2S-hydroxy-3S-(MC-Cal-His-amino)-4-cyclohexyl-butyl]-harnstoff
N-Isopropyl-N'-[2S-hydroxy-3S-(BOC-Hph-His-amino)-4-cyclohexyl-butyl]-harnstoff
N-Isopropyl-N'-[2S-hydroxy-3S-(MC-Hph-His-amino)-4-cyclohexyl-butyl]-harnstoff
N-Isopropyl-N'-[2S-hydroxy-3S-(BOC-αNal-His-amino)-4-cyclohexyl-butyl]-harnstoff
N-Isopropyl-N'-[2S-hydroxy-3S-(MC-αNal-His-amino)-4-cyclohexyl-butyl]-harnstoff
N-Isopropyl-N'-[2S-hydroxy-3s-(BOC-βNal-His-amino)-4-cyclohexyl-butyl]-harnstoff
N-Isopropyl-N'-[2S-hydroxy-3S-(MC-βNal-His-amino)-4-cyclohexyl-butyl]-harnstoff
N-Isopropyl-N'-[2S-hydroxy-3S-(BOC-Trp-His-amino)-4-cyclohexyl-butyl]-harnstoff
N-Isopropyl-N'-[2S-hydroxy-3S-(MC-Trp-His-amino)-4-cyclohexyl-butyl]-harnstoff
N-Isopropyl-N'-[2S-hydroxy-3S-(BOC-Tyr-His-amino)-4-cyclohexyl-butyl]-harnstoff
N-Isopropyl-N'-[2S-hydroxy-3S-(MC-Tyr-His-amino)-4-cyclohexyl-butyl]-harnstoff

N-Isopentyl-N'-[2S-hydroxy-3S-(BOC-Cal-His-amino)-4-cyclohexyl-butyl]-harnstoff
N-Isopentyl-N'-[2S-hydroxy-3S-(MC-Cal-His-amino)-4-cyclohexyl-butyl]-harnstoff
N-Isopentyl-N'-[2S-hydroxy-3S-(BOC-Hph-His-amino)-4-cyclohexyl-butyl]-harnstoff
N-Isopentyl-N'-[2S-hydroxy-3S-(MC-Hph-His-amino)-4-cyclohexyl-butyl]-harnstoff
N-Isopentyl-N'-[2S-hydroxy-3S-(BOC-αNal-His-amino)-4-cyclohexyl-butyl]-harnstoff
N-Isopentyl-N'-[2S-hydroxy-3S-(MC-αNal-His-amino)-4-cyclohexyl-butyl]-harnstoff
N-Isopentyl-N'-[2S-hydroxy-3S-(BOC-βNal-His-amiono)-4-cyclohexyl-butyl]-harnstoff
N-Isopentyl-N'-[2S-hydroxy-3S-(MC-βNal-His-amino)-4-cyclohexyl-butyl]-harnstoff
N-Isopentyl-N'-[2S-hydroxy-3S-(BOC-Trp-His-amino)-4-cyclohexyl-butyl]-harnstoff
N-Isopentyl-N'-[2S-hydroxy-3S-(MC-Trp-His-amino)-4-cyclohexyl-butyl]-harnstoff
N-Isopentyl-N'-[2S-hydroxy-3S-(BOC-Tyr-His-amino)-4-cyclohexyl-butyl]-harnstoff
N-Isopentyl-N'-[2S-hydroxy-3S-(MC-Tyr-His-amino)-4-cyclohexyl-butyl]-harnstoff
N-(2S-Hydroxy-3S-MC-Phe-His-amino-4-cyclohexyl-butyl)-phosphorsäure-monoamid-diethylester.

Beispiel 3

Analog Beispiel 2 erhält man aus N-[2S-Hydroxy-3S-MC-Phe-(imi-BOM-His)-amino-4-cyclohexyl-butyl]-carbaminsäureisopropylester [F. 101-105°; erhältlich durch Reaktion von 1-Amino-3S-BOC-amino-4-cyclohexyl-butan-2S-ol mit Chlorameisensäureisopropylester zu N-[2S-Hydroxy-3S-BOC-amino-4-cyclohexyl-butyl]-carbaminsäureisopropylester, Abspaltung der BOC-Gruppe, Reaktion mit BOC-(imi-BOM-His)-OH zu N-[2S-Hydroxy-3S-BOC-(imi-BOM-His)-amino-4-cyclohexyl-butyl]-carbaminsäureisopropylester, erneute Abspaltung der BOC-Gruppe und Reaktion mit MC-Phe-OH] den N-[2S-Hydroxy-3S-(MC-Phe-His-amino)-4-cyclohexyl-butyl]-carbaminsäureisopropylester, F. 90-92°.

Analog erhält man aus N-[2S-Hydroxy-3S-BOC-Phe-(imi-BOM-His)-amino-4-cyclohexyl-butyl]-carbaminsäureisopropylester (F. 119-122°) den N-[2S-Hydroxy-3S-BOC-Phe-His-amino-4-cyclohexyl-butyl]-carbaminsäureisopropylester, F. 170°.

Analog erhält man:
N-[2S-Hydroxy-3S-(ETOC-Phe-His-amino)-4-cyclohexyl-butyl]-carbaminsäureethylester
N-[2S-Hydroxy-3S-(IPOC-Phe-His-amino)-4-cyclohexyl-butyl]-carbaminsäureethylester
N-[2S-Hydroxy-3S-(BOC-Phe-His-amino)-4-cyclohexyl-butyl]-carbaminsäureethylester

N-[2S-Hydroxy-3S-(ETNC-Phe-His-amino)-4-cyclohexyl-butyl]-carbaminsäureethylester
N-[2S-Hydroxy-3S-(IPNC-Phe-His-amino)-4-cyclohexyl-butyl]-carbaminsäureethylester
N-[2S-Hydroxy-3S-(MC-Phe-His-amino)-4-cyclohexyl-butyl]-carbaminsäureethylester
N-[2S-Hydroxy-3S-(ETOC-Phe-His-amino)-4-cyclohexyl-butyl]-carbaminsäureisopropylester
N-[2S-Hydroxy-3S-(IPOC-Phe-His-amino)-4-cyclohexyl-butyl]-carbaminsäureisopropylester
N-[2S-Hydroxy-3S-(ETNC-Phe-His-amino)-4-cyclohexyl-butyl]-carbaminsäureisopropylester
N-[2S-Hydroxy-3S-(IPNC-Phe-His-amino)-4-cyclohexyl-butyl]-carbaminsäureisopropylester
N-[2S-Hydroxy-3S-(N,N-dimethylcarbamoyl-Phe-His-amino)-4-cyclohexyl-butyl]-carbaminsäureisopropylester
N-[2S-Hydroxy-3S-(N,N-dimethylcarbamoyl-Phe-His-amino)-4-cyclohexyl-butyl]-carbaminsäureisopropylester
N-[2S-Hydroxy-3S-(isopropylsulfonyl-Phe-His-amino)-4-cyclohexyl-butyl]-carbaminsäureisopropylester
N-[2S-Hydroxy-3S-(morpholinosulfonyl-Phe-His-amino)-4-cyclohexyl-butyl]-carbaminsäureisopropylester
N-[2S-Hydroxy-3S-(ETOC-Phe-His-amino)-4-cyclohexyl-butyl]-carbaminsäureisobutylester
N-[2S-Hydroxy-3S-(IPOC-Phe-His-amino)-4-cyclohexyl-butyl]-carbaminsäureisobutylester
N-[2S-Hydroxy-3S-(BOC-Phe-His-amino)-4-cyclohexyl-butyl]-carbaminsäureisobutylester, F. 147-149°
N-[2S-Hydroxy-3S-(ETNC-Phe-His-amino)-4-cyclohexyl-butyl]-carbaminsäureisobutylester
N-[2S-Hydroxy-3S-(IPNC-Phe-His-amino)-4-cyclohexyl-butyl]-carbaminsäureisobutylester
N-[2S-Hydroxy-3S-(MC-Phe-His-amino)-4-cyclohexyl-butyl]-carbaminsäureisobutylester
N-[2S-Hydroxy-3S-(ETOC-Phe-His-amino)-4-cyclohexyl-butyl]-carbaminsäureisopentylester
N-[2S-Hydroxy-3S-(IPOC-Phe-His-amino)-4-cyclohexyl-butyl]-carbaminsäureisopentylester
N-[2S-Hydroxy-3S-(BOC-Phe-His-amino)-4-cyclohexyl-butyl]-carbaminsäureisopentylester
N-[2S-Hydroxy-3S-(ETNC-Phe-His-amino)-4-cyclohexyl-butyl]-carbaminsäureisopentylester
N-[2S-Hydroxy-3S-(IPNC-Phe-His-amino)-4-cyclohexyl-butyl]-carbaminsäureisopentylester
N-[2S-Hydroxy-3S-(MC-Phe-His-amino)-4-cyclohexyl-butyl]-carbaminsäureisopentylester
N-[2S-Hydroxy-3S-(ETOC-Phe-His-amino)-4-cyclohexyl-butyl]-carbaminsäurecyclohexylester
N-[2S-Hydroxy-3S-(IPOC-Phe-His-amino)-4-cyclohexyl-butyl]-carbaminsäurecyclohexylester
N-[2S-Hydroxy-3S-(BOC-Phe-His-amino)-4-cyclohexyl-butyl]-carbaminsäurecyclohexylester
N-[2S-Hydroxy-3S-(ETNC-Phe-His-amino)-4-cyclohexyl-butyl]-carbaminsäurecyclohexylester
N-[2S-Hydroxy-3S-(IPNC-Phe-His-amino)-4-cyclohexyl-butyl]-carbaminsäurecyclohexylester
N-[2S-Hydroxy-3S-(MC-Phe-His-amino)-4-cyclohexyl-butyl]-carbaminsäurecyclohexylester
N-[2S-Hydroxy-3S-(ETOC-Phe-His-amino)-4-cyclohexyl-butyl]-carbaminsäurebenzylester
N-[2S-Hydroxy-3S-(IPOC-Phe-His-amino)-4-cyclohexyl-butyl]-carbaminsäurebenzylester
N-[2S-Hydroxy-3S-(BOC-Phe-His-amino)-4-cyclohexyl-butyl]-carbaminsäurebenzylester
N-[2S-Hydroxy-3S-(ETNC-Phe-His-amino)-4-cyclohexyl-butyl]-carbaminsäurebenzylester
N-[2S-Hydroxy-3S-(IPNC-Phe-His-amino)-4-cyclohexyl-butyl]-carbaminsäurebenzylester
N-[2S-Hydroxy-3S-(MC-Phe-His-amino)-4-cyclohexyl-butyl]-carbaminsäurebenzylester

Beispiel 4

Analog Beispiel 2 erhält man aus N-(1-Methoxycarbonyl-3-methyl-butyl)-N'-[2S-hydroxy-3S-(BOC-Phe-(imi-BOM-His)-amino)-4-cyclohexyl-butyl]-harnstoff [F. 177-179°; erhältlich durch Reaktion von 1-Amino-3S-BOC-amino-4-cyclohexylbutan-2S-ol mit 1S-Methoxycarbonyl-3-methyl-butyl-isocyanat in THF (2 Std. bei 20°) zu N-(1S-Methoxycarbonyl-3-methyl-butyl)-N'-(2S-hydroxy-3S-BOC-amino-4-cyclohexylbutyl)-harnstoff (F. 128-129°), Abspaltung der BOC-Gruppe, Reaktion mit BOC-(imi-BOM-His)-OH zu N-(1S-Methoxycarbonyl-3-methylbutyl)-N'-[2S-hydroxy-3S-BOC-(imi-BOM-His)-amino-4-cyclohexyl-butyl]-harnstoff (F. 73°), erneute Abspaltung der BOC-Gruppe und Reaktion mit BOC-Phe-OH] den N-(1S-Methoxycarbonyl-3-methyl-butyl)-N'-[2S-hydroxy-3S-(BOC-Phe-His-amino)-4-cyclohexyl-butyl]-harnstoff, F. 180-182° (Zersetzung).

Analog erhält man aus N-(1S-Methoxycarbonyl-3-methylbutyl)-N'-[2R-hydroxy-3S-(BOC-Phe-(imi-BOM-His)-amino-4-cyclohexyl-butyl]-harnstoff (F. 87-88°) den N-(1S-Methoxycarbonyl-3-methyl-butyl)-N'-[2R-hydroxy-3S-(BOC-Phe-His-amino)-4-cyclohexyl-butyl]-harnstoff, F. 171-172°.

Analog erhält man:
N-(1S-Carbamoyl-3-methyl-butyl)-N'-[2S-hydroxy-3S-(BOC-Phe-His-amino)-4-cyclohexyl-butyl]-harnstoff.

Beispiel 5

Analog Beispiel 2 erhält man aus N-[1S-N-(3-Amino-5,6-dimethyl-pyrazin-2-ylmethyl)-carbamoyl-3-methyl-butyl]-N'-[2S-hydroxy-3S-BOC-Phe-(imi-BOM-His)-amino-4-cyclohexyl-butyl]-harnstoff [F. 211-213°; erhältlich durch Verseifung von N-(1S-Methoxycarbonyl-3-methyl-butyl)-N'-(2S-hydroxy-3S-BOC-amino-4-cyclohexyl-butyl)-harnstoff mit 2 n NaOH in Dioxan zu N-(1S-Carboxy-3-methyl-butyl)-N'-(2S-hydroxy-3S-BOC-amino-4-cyclohexyl-butyl)-harnstoff (F. 145-147°), Umsetzung mit 2-Aminomethyl-3-amino-5,6-dimethyl-pyrazin/DCCI/HOBt zu N-[1S-N-(3-Amino-5,6-dimethyl-pyrazin-2-ylmethyl)-carbamoyl-3-methyl-butyl]-N'-(2S-hydroxy-3S-BOC-amino-4-cyclohexyl-butyl)-harnstoff (F. 105-106°), Abspaltung der BOC-Gruppe, Reaktion mit BOC-(imi-BOM-His)-OH zu N-[1S-N-(3-Amino-5,6-dimethyl-pyrazin-2-yl-methyl)-carbamoyl-3-methyl-butyl]-N'-[2S-hydroxy-3S-BOC-(imi-BOM-His)-amino-4-cyclohexyl-butyl]-harnstoff (F. 184-186°), erneute Abspaltung der BOC-Gruppe und Reaktion mit BOC-Phe-OH] den N-[1S-N-(3-Amino-5,6-dimethyl-pyrazin-2-ylmethyl)-carbamoyl-3-methyl-butyl]-N'-[2S-hydroxy-3S-(BOC-Phe-His-amino)-4-cyclohexyl-butyl]-harnstoff, F. 177-179°.

Analog erhält man:
N-[1S-N-(5-Tetrazolyl-methyl)-carbamoyl-3-methylbutyl]-N'-[2S-hydroxy-3S-(BOC-Phe-His-amino)-4-cyclohexyl-butyl]-harnstoff
N-[1S-N-(2-Pyridyl-methyl)-carbamoyl-3-methylbutyl]-N'-[2S-hydroxy-3S-(BOC-Phe-His-amino)-4-cyclohexyl-butyl]-harnstoff
N-[1S-N-(3-Pyridyl-methyl)-carbamoyl-3-methylbutyl]-N'-[2S-hydroxy-3S-(BOC-Phe-His-amino)-4-cyclohexyl-butyl]-harnstoff
N-[1S-N-(2-(4-Pyridyl)-ethyl)-carbamoyl-3-methylbutyl]-N'-[2S-hydroxy-3S-(BOC-Phe-His-amino)-4-cyclohexyl-butyl]-harnstoff
N-[1S-N-(2-Hydroxy-4,6-dimethyl-3-pyridylmethyl)-carbamoyl-3-methylbutyl]-N'-[2S-hydroxy-3S-(BOC-Phe-His-amino)-4-cyclohexyl-butyl]-harnstoff
N-[1S-N-(2-Methyl-4-amino-5-pyrimidylmethyl)-carbamoyl-3-methylbutyl]-N'-[2S-hydroxy-3S-(BOC-Phe-His-amino)-4-cyclohexyl-butyl]-harnstoff.

Beispiel 6

Analog Beispiel 2 erhält man aus N-[2S-Hydroxy-3S-(BOC-Phe-(imi-BOM-His)-amino)-4-cyclohexyl-butyl]-isopropylsulfonamid [erhältlich durch Reaktion von 2S-BOC-amino-3-cyclohexyl-propanal mit Trimethylsilylcyanid/ZnJ$_2$ in THF bei 0° zu 2-Hydroxy-3S-BOC-amino-4-cyclohexyl-butyronitril (Gemisch der 2S-und 2R-Epimeren), Umsetzung mit Dimethyl-tert.-butyl-silyl-chlorid/Imidazol in DMF bei 20° und anschließende chromatographische Trennung zu 2S-Dimethyl-tert.-butyl-silyloxy-3S-BOC-amino-4-cyclohexyl-butyronitril (F. 83-85°; daneben das 2R-Epimere, F. 113-114°), Reaktion mit Raney-Ni in 10%igem methanolischem NH$_3$ bei 5 bar und 60° zu 2S-Dimethyl-tert.-butylsilyloxy-3S-BOC-amino-4-cyclohexyl-butylamin (Öl), Umsetzung mit Tetrabutylammoniumfluorid in THF bei 20° zu 1-Amino-3S-BOC-amino-4-cyclohexyl-butan-2S-ol, Reaktion mit Isopropylsulfonylchlorid in CHCl$_3$/Pyridin bei 5° zu N-(2S-Hydroxy-3S-BOC-amino-4-cyclohexyl-butyl-isopropylsulfonamid (F. 156°) und Aufbau der Peptid-Seitenkette analog Beispiel 2] das N-[2S-Hydroxy-3S-(BOC-Phe-His-amino)-4-cyclohexyl-butyl]-isopropylsulfonamid, F. 117° (Zersetzung).

Analog erhält man:
N-[2S-Hydroxy-3S-(MC-Phe-His-amino)-4-cyclohexyl-butyl]-thioharnstoff
N-[2S-Hydroxy-3S-(BOC-Phe-His-amino)-4-cyclohexyl-butyl]-methylsulfonamid, F. 170°
N-[2S-Hydroxy-3S-(BOC-Phe-His-amino)-4-cyclohexyl-butyl]-ethylsulfonamid
N-[2S-Hydroxy-3S-(BOC-Phe-His-amino)-4-cyclohexyl-butyl]-cyclohexylsulfonamid
N-[2S-Hydroxy-3S-(BOC-Phe-His-amino)-4-cyclohexyl-butyl]-phenylsulfonamid
N-[2S-Hydroxy-3S-(BOC-Phe-His-amino)-4-cyclohexyl-butyl]-sulfamid
4-[N-[2S-Hydroxy-3S-(BOC-Phe-His-amino)-4-cyclohexyl-butyl]-aminosulfonyl]-morpholin
N-[2S-Hydroxy-3S-(IPOC-Phe-His-amino)-4-cyclohexyl-butyl]-isopropylsulfonamid, F. 156°
N-[2S-Hydroxy-3S-(IPNC-Phe-His-amino)-4-cyclohexyl-butyl]-isopropylsulfonamid
N-[2S-Hydroxy-3S-(MC-Phe-His-amino)-4-cyclohexyl-butyl]-isopropylsulfonamid.

Beispiel 7

Eine Lösung von 4,77 g N-[1S-N-(3-Amino-5,6-dimethyl-pyrazin-2-ylmethyl)-carbamoyl-3-methyl-butyl]-N'-(2S-hydroxy-3S-amino-4-cyclohexyl-butyl)-harnstoff in 60 ml Dichlormethan wird mit 1,01 g N-Methylmorpholin versetzt. Unter Rühren gibt man 3,22 g BOC-Phe-Gly-OH, 1,35 g HOBt und eine Lösung von 2,06 g DCCI in 50 ml Dichlormethan hinzu, rührt 14 Std. bei 4°, filtriert den ausgefallenen Dicyclohexylharnstoff ab, dampft das Filtrat ein, arbeitet wie üblich auf und erhält N-[1S-N-(3-Amino-5,6-dimethyl-pyrazin-2-ylmethyl)-carbamoyl-3-methylbutyl]-N'-(2S-hydroxy-3S-BOC-Phe-Gly-amino-4-cyclohexyl-butyl)-harnstoff, F. 132° (Zersetzung).

Analog erhält man:
N-Isopropyl-N'-[2S-hydroxy-3S-(BOC-Phe-Abu-amino)-4-cyclohexyl-butyl]-harnstoff
N-Isopropyl-N'-[2S-hydroxy-3S-(MC-Phe-Abu-amino)-4-cyclohexyl-butyl]-harnstoff
N-Isopropyl-N'-[2S-hydroxy-3S-(BOC-Phe-Gly-amino)-4-cyclohexyl-butyl]-harnstoff
N-Isopropyl-N'-[2S-hydroxy-3S-(MC-Phe-Gly-amino)-4-cyclohexyl-butyl]-harnstoff
N-Isopropyl-N'-[2S-hydroxy-3S-(BOC-Phe-N-Me-His-amino)-4-cyclohexyl-butyl]-harnstoff
N-Isopropyl-N'-[2S-hydroxy-3S-(MC-Phe-N-Me-His-amino)-4-cyclohexyl-butyl]-harnstoff
N-Isopropyl-N'-[2S-hydroxy-3S-(BOC-Phe-Leu-amino)-4-cyclohexyl-butyl]-harnstoff
N-Isopropyl-N'-[2S-hydroxy-3S-(MC-Phe-Leu-amino)-4-cyclohexyl-butyl]-harnstoff
N-Isopropyl-N'-[2S-hydroxy-3S-(BOC-Phe-Met-amino)-4-cyclohexyl-butyl]-harnstoff
N-Isopropyl-N'-[2S-hydroxy-3S-(MC-Phe-Met-amino)-4-cyclohexyl-butyl]-harnstoff
N-Isopropyl-N'-[2S-hydroxy-3S-(BOC-Phe-Nle-amino)-4-cyclohexyl-butyl]-harnstoff
N-Isopropyl-N'-[2S-hydroxy-3S-(MC-Phe-Nle-amino)-4-cyclohexyl-butyl]-harnstoff
N-Isopropyl-N'-[2S-hydroxy-3S-(BOC-Phe-Phe-amino)-4-cyclohexyl-butyl]-harnstoff
N-Isopropyl-N'-[2S-hydroxy-3S-(MC-Phe-Phe-amino)-4-cyclohexyl-butyl]-harnstoff
N-Isopropyl-N'-[2S-hydroxy-3S-(BOC-Phe-(3-Pyr)-amino)-4-cyclohexyl-butyl]-harnstoff
N-Isopropyl-N'-[2S-hydroxy-3S-(MC-Phe-(3-Pyr)-amino)-4-cyclohexyl-butyl]-harnstoff
N-Isopropyl-N'-[2S-hydroxy-3S-(BOC-Phe-Trp-amino)-4-cyclohexyl-butyl]-harnstoff
N-Isopropyl-N'-[2S-hydroxy-3S-(MC-Phe-Trp-amino)-4-cyclohexyl-butyl]-harnstoff
N-Isopentyl-N'-[2S-hydroxy-3S-(BOC-Phe-Abu-amino)-4-cyclohexyl-butyl]-harnstoff
N-Isopentyl-N'-[2S-hydroxy-3S-(MC-Phe-Abu-amino)-4-cyclohexyl-butyl]-harnstoff
N-Isopentyl-N'-[2S-hydroxy-3S-(BOC-Phe-Gly-amino)-4-cyclohexyl-butyl]-harnstoff
N-Isopentyl-N'-[2S-hydroxy-3S-(MC-Phe-Gly-amino)-4-cyclohexyl-butyl]-harnstoff
N-Isopentyl-N'-[2S-hydroxy-3S-(BOC-Phe-N-Me-His-amino)-4-cyclohexyl-butyl]-harnstoff
N-Isopentyl-N'-[2S-hydroxy-3S-(MC-Phe-N-Me-His-amino)-4-cyclohexyl-butyl]-harnstoff
N-Isopentyl-N'-[2S-hydroxy-3S-(BOC-Phe-Leu-amino)-4-cyclohexyl-butyl]-harnstoff
N-Isopentyl-N'-[2S-hydroxy-3S-(MC-Phe-Leu-amino)-4-cyclohexyl-butyl]-harnstoff
N-Isopentyl-N'-[2S-hydroxy-3S-(BOC-Phe-Met-amino)-4-cyclohexyl-butyl]-harnstoff
N-Isopentyl-N'-[2S-hydroxy-3S-(MC-Phe-Met-amino)-4-cyclohexyl-butyl]-harnstoff
N-Isopentyl-N'-[2S-hydroxy-3S-(BOC-Phe-Nle-amino)-4-cyclohexyl-butyl]-harnstoff
N-Isopentyl-N'-[2S-hydroxy-3S-(MC-Phe-Nle-amino)-4-cyclohexyl-butyl]-harnstoff
N-Isopentyl-N'-[2S-hydroxy-3S-(BOC-Phe-Phe-amino)-4-cyclohexyl-butyl]-harnstoff
N-Isopentyl-N'-[2S-hydroxy-3S-(MC-Phe-Phe-amino)-4-cyclohexyl-butyl]-harnstoff
N-Isopentyl-N'-[2S-hydroxy-3S-(BOC-Phe-(3-Pyr)-amino)-4-cyclohexyl-butyl]-harnstoff
N-Isopentyl-N'-[2S-hydroxy-3S-(MC-Phe-(3-Pyr)-amino)-4-cyclohexyl-butyl]-harnstoff
N-Isopentyl-N'-[2S-hydroxy-3S-(BOC-Phe-Trp-amino)-4-cyclohexyl-butyl]-harnstoff
N-Isopentyl-N'-[2S-hydroxy-3S-(MC-Phe-Trp-amino)-4-cyclohexyl-butyl]-harnstoff.

Beispiel 8

Analog Beispiel 4 erhält man aus BOC-Phe-OH und N-Isopentyl-N'-[2S-hydroxy-3S-(H-Gly-amino)-4-cyclohexyl-butyl]-harnstoff [erhältlich durch Reaktion von BOC-Gly-OH mit N-Isopentyl-N'-(2S-hydroxy-3S-amino-4-cyclohexyl-butyl)-harnstoff zur 3S-BOC-Gly-amino-verbindung und nachfolgende Abspaltung der BOC-Gruppe] den N-Isopentyl-N'-[2S-hydroxy-3S-(BOC-Phe-Gly-Amino)-4-cyclohexyl-butyl]-harnstoff.

Beispiel 9

Eine Lösung von 1 g N-Isopentyl-N'-(2S-hydroxy-3S-BOC-Phe-His-amino-4-cyclohexyl-butyl)-harnstoff in 20 ml 4 n HCl in Dioxan wird 30 Min. bei 20° gerührt und dann eingedampft. Man erhält N-Isopentyl-N'-(2S-hydroxy-3S-H-Phe-His-amino-4-cyclohexyl-butyl)-harnstoff.
Analog erhält man durch Spaltung der entsprechenden N-BOC-Derivate:
N-(1-Methoxycarbonyl-3-methylbutyl)-N'-(2R-hydroxy-3S-H-Phe-His-amino-4-cyclohexyl-butyl)-harnstoff.
N-(1-Methoxycarbonyl-3-methylbutyl)-N'-(2S-hydroxy-3S-H-Phe-His-amino-4-cyclohexyl-butyl)-harnstoff
N-[1-N-(3-Amino-5.6-dimethylpyrazin-2-ylmethyl)-carbamoyl-3-methyl-butyl]-N'-(2S-hydroxy-3S-H-Phe-Gly-amino-4-cyclohexyl-butyl)-harnstoff
N-sek.-Butyl-N'-(2S-hydroxy-3S-H-Phe-His-amino-4-cyclohexyl-butyl)-harnstoff
N-[1-N-(3-Amino-5.6-dimethylpyrazin-2-ylmethyl)-carbamoyl-3-methyl-butyl]-N'-(2S-hydroxy-3S-H-Phe-amino-4-cyclohexyl-butyl)-harnstoff
N'-(2S-Hydroxy-3S-H-Phe-His-amino-4-cyclohexyl-butyl)-carbaminsäure-isopropylester
N-Isopropyl-N'-(2S-hydroxy-3S-H-Phe-His-amino-4-phenylbutyl)-harnstoff
N-Isopropyl-N'-(2S-hydroxy-3S-H-Phe-His-amino-4-cyclohexylbutyl)-harnstoff

Beispiel 10

Man löst 1 g N-Isopentyl-N'-(2S-hydroxy-3S-CBZ-Phe-His-amino-4-cyclohexyl-butyl)-harnstoff in 15 ml Ethanol, hydriert 3 Std. an 0,5 g 10%ig. Pd-C bei 20° und 1 bar, filtriert, dampft ein und erhält nach chromatographischer Reinigung N-Isopentyl-N'-(2S-hydroxy-3-H-Phe-His-amino-4-cyclohexyl-butyl)-harnstoff.

Beispiel 11

Analog Beispiel 2 erhält man durch Hydrogenolyse der entsprechenden imi-BOM-His-Verbindungen:

N-[2S-Hydroxy-3S-(BOC-Phe-His-amino)-4-cyclohexyl-butyl]-benzylsulfonamid

N-[2S-Hydroxy-3S-(BOC-Phe-His-amino)-4-cyclohexyl-butyl]-isobutylsulfonamid

N-[2S-Hydroxy-3S-(BOC-Phe-His-amino)-4-cyclohexyl-butyl]-butylsulfonamid

N-[2S-Hydroxy-3S-(BOC-αNal-His-amino)-4-cyclohexyl-butyl]-isobutylsulfonamid

4-[N-(2S-Hydroxy-3S-(tert.-butylacetyl-Phe-His-amino)-4-cyclohexyl-butyl)-aminosulfonyl]-morpholin

4-[N-(2S-Hydroxy-3S-(morpholinoacetyl-Phe-His-amino)-4-cyclohexyl-butyl)-aminosulfonyl]-morpholin

N-[2S-Hydroxy-3S-(morpholinoacetyl-Phe-His-amino)-4-cyclohexyl-butyl)-isobutylsulfonamid

N-[2S-Hydroxy-3S-(morpholinoacetyl-Phe-N-Me-His-amino)-4-cyclohexyl-butyl)-benzylsulfonamid

N-[2S-Hydroxy-3S-(IPOC-Phe-N-Me-His-amino)-4-cyclohexyl-butyl)-benzylsulfonamid.

Die nachstehenden Beispiele betreffen pharmazeutische Zubereitungen.

Beispiel A: Injektionsgläser

Eine Lösung von 100 g N-Isopentyl-N'-[2S-hydroxy-3S-(BOC-Phe-His-amino)-4-cyclohexyl-butyl]-harnstoff und 5 g Dinatriumhydrogenphosphat in 3 l zweifach destilliertem Wasser wird mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 500 mg Wirkstoff.

Beispiel B: Suppositorien

Man schmilzt ein Gemisch von 500 g N-sek.-Butyl-N'-[2S-hydroxy-3S-(BOC-Phe-His-amino)-4-cyclohexyl-butyl]-harnstoff mit 100 g Sojalecithin und 1400 g Kakaobutter, gießt in Formen und läßt erkalten. Jedes Suppositorium enthält 500 mg Wirkstoff.

**Ansprüche**

1. Aminosäurederivate der Formel I
X-Z-NR$^2$-CHR$^3$-CHOH-(CH$_2$)$_n$-NR$^4$-E-Y    I
worin
X  H, R$^1$-O-C$_m$H$_{2m}$-CO-, R$^1$-C$_m$H$_{2m}$-O-CO-, R$^1$-C$_m$H$_{2m}$-CO-, R$^1$-SO$_2$-oder (R$^1$-C$_m$H$_{2m}$)-L(R$^1$-C$_p$H$_{2p}$)-C$_r$H$_{2r}$-CO-,
Z  1 bis 4 peptidartig miteinander verbundene Aminosäurereste ausgewählt aus der Gruppe bestehend aus Abu, Ada, Ala, Arg, Asn, Asp, Bia, Cal, Dab, Gln, Glu, Gly, His, Hph, N(im)-Alkyl-His, Ile, Leu, tert.-Leu, Lys, Met, αNal, βNal, Nbg, Nle, Orn, Phe, Pro, Pyr, Ser, Thr, Tic, Trp, Tyr und Val,
E  -CONH-, -CSNH-, -COO-, -SO$_2$-, -SO$_2$NH-oder -PO(OA)-O-,
Y  R$^5$, -(CHR$^5$)$_s$-COOR$^6$ oder -(CHR$^5$)$_s$-CONR$^7$R$^8$,
R$^1$, R$^3$, R$^6$, R$^7$ und R$^8$ jeweils H, A, Ar, Ar-alkyl, Het, Het-alkyl, unsubstituiertes oder ein-oder mehrfach durch A, AO und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,
R$^2$ und R$^4$ jeweils H oder A,
R$^5$  H, A, Ar, Ar-alkyl, Cycloalkyl mit 3-7 C-Atomen oder Cycloalkylalkyl mit 4-11 C-Atomen,
L  CH oder N,
m, p und r jeweils 0, 1, 2, 3, 4 oder 5,
n  1 oder 2,
s  0 oder 1,
Ar  unsubstituiertes oder ein-oder mehrfach durch A, AO, Hal, CF$_3$, OH und/oder NH$_2$ substituiertes Phenyl oder unsubstituiertes Naphthyl,
Het  einen gesättigten oder ungesättigten 5-oder 6-gliedrigen heterocyclischen Rest mit 1-4 N-, O-und/oder S-Atomen, der mit einem Benzolring kondensiert und/oder ein-oder mehrfach durch A, AO, Hal, CF$_3$, HO, O$_2$N, Carbonylsauerstoff, H$_2$N, HAN, A$_2$N, AcNH, AS, ASO, ASO$_2$, AOOC, CN, H$_2$NCO, H$_2$NSO$_2$, ASO$_2$NH, Ar, Ar-alkenyl, Hydroxyalkyl und/oder Aminoalkyl mit jeweils 1-8 C-Atomen substituiert sein kann und/oder dessen N-und/oder S-Heteroatome auch oxidiert sein können,
Hal  F, Cl, Br oder J,
Ac  A-CO-, Ar-CO-oder A-NH-CO-,
-alkyl-eine Alkylengruppe mit 1-8 C-Atomen und
A  Alkyl mit 1-8 C-Atomen bedeuten,

E-Y auch Pyrrolidinocarbonyl, Piperidinocarbonyl, Morpholinocarbonyl, Pyrrolidinosulfonyl, Piperidinosulfonyl oder Morpholinosulfonyl bedeuten kann, worin ferner an Stelle einer oder mehrerer -NH-CO-Gruppen auch eine oder mehrere -NA-CO-Gruppen stehen können,
sowie deren Salze.

2. a) N-Isopropyl-N'-(2S-hydroxy-3S-IPOC-Phe-His-amino-4-cyclohexyl-butyl)-harnstoff;

b) N-Isopropyl-N'-(2S-hydroxy-3S-BOC-Phe-His-amino-4-cyclohexyl-butyl)-harnstoff;

c) N-Isopropyl-N'-(2S-hydroxy-3S-BOC-Phe-His-amino-4-phenyl-butyl)-harnstoff;

d) N-sek.-Butyl-N'-(2S-hydroxy-3S-BOC-Phe-His-amino-4-cyclohexyl-butyl)-harnstoff;

e) N-Isopentyl-N'-(2S-hydroxy-3S-BOC-Phe-His-amino-4-cyclohexyl-butyl)-harnstoff;

f) N-(1S-Methoxycarbonyl-3-methylbutyl)-N'-(2S-hydroxy-3S-BOC-Phe-His-amino-4-cyclohexyl-butyl)-harnstoff;

g) N-(1S-Methoxycarbonyl-3-methylbutyl)-N'-(2R-hydroxy-3S-BOC-Phe-His-amino-4-cyclohexylbutyl)-harnstoff;

h) N-[1S-N-(3-Amino-5,6-dimethylpyrazin-2-ylmethyl)-carbamoyl-3-methylbutyl]-N'-(2S-hydroxy-3S-BOC-Phe-Gly-amino-4-cyclohexyl-butyl)-harnstoff;

i) N-[1S-N-(3-Amino-5,6-dimethylpyrazin-2-ylmethyl)-carbamoyl-3-methylbutyl]-N'-(2S-hydroxy-3S-BOC-Phe-His-amino-4-cyclohexyl-butyl)-harnstoff;

j) N-(2S-Hydroxy-3S-morpholinocarbonyl-Phe-His-amino-4-cyclohexyl-butyl)-carbaminsäure-isopropylester;

k) N-(2S-Hydroxy-3S-BOC-Phe-His-amino-4-cyclohexyl-butyl)-carbaminsäure-isopropylester.

3. Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt oder daß man eine Verbindung, die der Formel I entspricht, aber an Stelle von H-Atomen eine oder mehrere zusätzliche C-C-und/oder C-N-und/oder C-O-Bindungen und/oder O-Atome enthält, reduziert
oder daß man eine Carbonsäure der Formel II
X-G$^1$-OH    II
worin G$^1$ (a) Z$^1$,
(b) Z
bedeutet
mit einer Aminoverbindung der Formel III
H-G$^2$    III
worin G$^2$ (a) -Z$^2$-NR$^2$-CHR$^3$-CHOH-(CH$_2$)$_n$-NR$^4$-E-Y,
(b) -NR$^2$-CHR$^3$-CHOH-(CH$_2$)$_n$-NR$^4$-E-Y und
Z$^1$ + Z$^2$ zusammen Z bedeuten
umsetzt,
und daß man gegebenenfalls in einer Verbindung der Formel I eine funktionell abgewandelte Amino-und/oder Hydroxygruppe durch Behandeln mit solvolysierenden oder hydrogenolysierenden Mitteln in Freiheit setzt und/oder einen Rest Y durch Behandeln mit veresternden, solvolysierenden, acylierenden oder amidierenden Mitteln in einen anderen Rest Y umwandelt und/oder eine Verbindung der Formel I durch Behandeln mit einer Säure in eines ihrer Salze überführt.

4. Verfahren zur Herstellung pharmazeutischer Zubereitungen, dadurch gekennzeichnet, daß man eine Verbindung der Formel I und/oder eines ihres physiologisch unbedenklichen Salze zusammen mit mindestens einem festen, flüssigen oder halbflüssigen Träger-oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verwendung von Verbindungen der Formel I oder von deren physiologisch unbedenklichen Salzen bei der Bekämpfung der reninabhängigen Hypertension oder des Hyperaldosteronismus.

7. Verwendung von Verbindungen der Formel I oder von deren physiologisch unbedenklichen Salzen zur Herstellung eines Arzneimittels.